(19) **Europäisches Patentamt / European Patent Office / Office européen des brevets**

(11) **EP 1 953 244 A1**

(12) **EUROPEAN PATENT APPLICATION**

(43) Date of publication:
**06.08.2008 Bulletin 2008/32**

(51) Int Cl.:
***C12Q 1/68*** (2006.01)

(21) Application number: **08007724.1**

(22) Date of filing: **09.01.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **09.01.2002 US 346952 P**

(62) Document number(s) of the earlier application(s) in accordance with Art. 76 EPC:
**03700442.1 / 1 466 016**

(71) Applicants:
• **Nakamura, Yusuke**
**Tokyo 108-8639 (JP)**

• **Ohnishi, Yasuyuki**
**Kawasaki,**
**Kanagawa 216-0001 (JP)**

(72) Inventor: **Katagiri, Toyomasa**
**Tokyo 108-8639 (JP)**

(74) Representative: **Glawe, Delfs, Moll**
**Patentanwälte**
**Rothenbaumchaussee 58**
**20148 Hamburg (DE)**

Remarks:
This application was filed on 21-04-2008 as a divisional application to the application mentioned under INID code 62.

(54) **Cancer profiles**

(57) The present invention relates to genetic profiles and markers of cancers and provides systems and methods for screening drugs that are effective for specific patients and types of cancers, in particular, the present invention provides personalized treatment customized to an individual's cancer.

EP 1 953 244 A1

## Description

This Application claims priority to U.S. Provisional Patent application serial no. 60/346,952, filed 1/9/02, which is herein incorporated by reference in its entirety.

## FIELD OF THE INVENTION

The present invention relates to genetic profiles and markers of cancers and provides systems and methods for screening drugs that are effective for specific patients and types of cancers.

## BACKGROUND OF THE INVENTION

The efficacy of anti-cancer drugs varies widely among individual patients. A large proportion of cancer patients suffer adverse effects of chemotherapy while showing no effective response in terms of tumor regression. No prediction of effectiveness prior to treatment can be done at present, with a few exceptions such as tamoxifen treatment for patients with ER-positive breast cancer. Numerous investigators have attempted to establish a diagnostic method for predicting chemosensitivity, and a few markers have been identified (Furukawa et al., Clin Cancer Res. 1: 305-11 [1995]; Salonga et al., Clin Cancer Res. 6: 1322-7 [2000]; Ueda et al., J Biol Chem. 262: 505-8 [1987]; Turton et al., Oncogene. 20: 1300-6 [2001]). However, properties of cancer cells are determined by complicated interactions among all gene products expressed in cancer cells, and it is certain that many proteins, including enzymes involved in apoptosis, DNA repair, and metabolism and detoxification of drugs, affect individual responses. Hence, to distinguish responders from non-responders before starting treatment, i.e., to offer a "personalized" program of more effective chemotherapy and also to relieve patients from unnecessary side effects, a larger set of genes should be identified to serve as accurate predictive markers.

## SUMMARY OF THE INVENTION

The present invention provides genetic profiles and markers of cancers and provides systems and methods for screening drugs that are effective for specific patients and types of cancers.

Accordingly, in some embodiments, the present invention provides a method, comprising providing a sample from a subject diagnosed with cancer; and generating a drug sensitivity profile for said sample. In some embodiments, generating a drug sensitivity profile comprises detecting a level of expression of one or more drug sensitivity genes (e.g., including, but not limited to, ADAMTS2, MSH6, MAGEA6, RPS6KB1, PIK3CB, MDR3, MDR4, MDR9, OS4, KIAA1140, GCNT3, FARP1, PP1044, C11ORF15, KIAA0233, BZRP, PRPS1, HGFAC, TRPC4, LOC56889, FLJ20208, FLJ22833, PGLYRP, TNFRSF14, HLA-B, NPR3, DES, PCDH1, LLGL2, HMGCL, TSPAN, ANXA4, ABP1, ERP70, HSD17B2, FBP1, SIM1, LAF4, CRKL, TOB2, GDA, MMP7, PRSS8, CKS2, PGLYRP, KIF4A, PABPC1, FLJ21865, ETV4, EXTL3, PIK3C2A, DNMT3B, DKFZP566G1424, KIAA0026, CCNB1, GPC3, EVA1, FSTL3, MSLN, FLJ21935, DES, STK17A, KCNH2, DLK1, CPX, NRP1, PCTAIRE2BP, ELF3, AGR2, FLJ10849, ATP1B1, GPX2, SLI, AMPD1, OTC, MMP3, CPX, COX6A2, S100A4, LOC51315, MDS024, PCDH1, PGLYRP, NK4, TRAF2, ARHE, LOC51256, ITGA3, KIAA0971, KIAA1037, GABRA6, U1SNRNPBP, MYBL2, POLA2, TRH, KRTHB6, COL3A1, PDK3, NNAT, FLJ20510, FLJ20208, ANXA4, MMP7, ETS1, ABLIM, CPT1A, BARD1, OKL38, ATP1B1, TONDU, STC1, ABLIM, GCNT3, UCP3, SLC12A2, LOC51141, HSD17B2, ITIH2, INADL, ANXA4, RNASE6PL, CKS2, PABPC1, GJB1, CUTL1, SPTBN1, FLJ13881, LGALS4, C11ORF9, ARSE, LAMB3, MVP, UGT1A1, GLJ20053, CD74, BUB1B, CCND1, CCNE1, TOP2A, TYMS, ALDH1, and CYP3A5). In some embodiments, the drug sensitivity profile comprises drug sensitivity scores for one or more drugs. In some embodiments, the drugs are cancer chemotherapy drugs (e.g., including, but not limited to, 5FU, ACNU, ADR, CPM, DDP, MMC, MTX, VCR, and VLB). In some embodiments, the method further comprises the step of determining a treatment course of action based on the drug sensitivity profile. In some embodiments, the treatment course of action comprises a choice of cancer chemotherapy drugs for administration to the subject. In some embodiments, detecting the level of expression of one or more drug sensitivity genes comprises detecting the level of mRNA expressed from the drug sensitivity genes. In some embodiments, detecting the level of mRNA expressed from the drug sensitivity genes comprises exposing the mRNA to a nucleic acid probe complementary to the mRNA. In other embodiments, detecting the level of mRNA expressed from the drug sensitivity genes comprises performing an INVADER assay. In some further embodiments, detecting the level of expression of one or more drug sensitivity genes comprises detecting the level of polypeptide expressed from the drug sensitivity genes. In some embodiments, detecting the level of polypeptide expressed from the drug sensitivity genes comprises exposing the polypeptide to an antibody specific to the polypeptide and detecting the binding of the antibody to the polypeptide. In some embodiments, the subject comprises a human subject. In certain embodiments, the sample comprises tumor tissue.

The present invention additionally provides a kit for characterizing cancer in a subject, comprising a reagent capable of specifically detecting the level of expression of one or more drug sensitivity genes; and instructions for using the kit for characterizing cancer in the subject. In some embodiments, the drug sensitivity genes are selected from the group including, but not limited to, ADAMTS2, MSH6, MAGEA6, RPS6KB1, PIK3CB, MDR3, MDR4, MDR9, OS4, KIAA1140, GCNT3, FARP1, PP1044, C110RF15, KIAA0233, BZRP, PRPS1, HGFAC, TRPC4, LOC56889, FLJ20208, FLJ22833, PGLYRP, TNFRSF14, HLA-B, NPR3, DES, PCDH1, LLGL2, HMGCL, TSPAN, ANXA4, ABP1, ERP70, HSD17B2, FBP1, SIM1, LAF4, CRKL, TOB2, GDA, MMP7, PRSS8, CKS2, PGLYRP, KIF4A, PABPC1, FLJ21865, ETV4, EXTL3, PIK3C2A, DNMT3B, DKFZP566G1424, KIAA0026, CCNB1, GPC3, EVA1, FSTL3, MSLN, FLJ21935, DES, STK17A, KCNH2, DLK1, CPX, NRP1, PCTAIRE2BP, ELF3, AGR2, FLJ10849, ATP1B1, GPX2, SLI, AMPD1, OTC, MMP3, CPX, COX6A2, S100A4, LOC51315, MDS024, PCDH1, PGLYRP, NK4, TRAF2, ARHE, LOC51256, ITGA3, KIA.A0971, KIAA1037, GABRA6, U1SNRNPBP, MYBL2, POLA2, TRH, KRTHB6, COL3A1, PDK3, NNAT, FLJ20510, FLJ20208, ANXA4, MMP7, ETS1, ABLIM, CPT1A, BARD1, OKL38, ATP1B1, TONDU, STC1, ABLIM, GCNT3, UCP3, SLC12A2, LOC51141, HSD17B2, ITIH2, INADL, ANXA4, RNASE6PL, CKS2, PABPC1, GJB1, CUTL1, SPTBN1, FLJ13881, LGALS4, C11ORF9, ARSE, LAMB3, MVP, UGT1A1, GLJ20053, CD74, BUB1B, CCND1, CCNE1, TOP2A, TYMS, ALDH1, and CYP3A5. In some embodiments, the reagent comprises a nucleic acid probe complementary to an mRNA expressed from the drug sensitivity gene. In other embodiments, the reagent comprises an antibody that specifically binds to a polypeptide expressed from the drug sensitivity gene. In some embodiments, the instructions comprise instructions required by the United States Food and Drug Administration for use in *in vitro* diagnostic products.

The present invention further provides a method of screening compounds, comprising providing a cancer sample comprising a known drug sensitivity profile; a plurality of test compounds; and determining the effectiveness of the test compound in treating the cancer. In some embodiments, the drug sensitivity profile comprises the level of expression of one or more drug sensitivity genes (e.g., including, but not limited to, ADAMTS2, MSH6, MAGEA6, RPS6KB1, PIK3CB, MDR3, MDR4, MDR9, OS4, KIAA1140, GCNT3, FARP1, PP1044, C11ORF15, KIAA0233, BZRP, PRPS1, HGFAC, TRPC4, LOC56889, FLJ20208, FLJ22833, PGLYRP, TNFRSF14, HLA-B, NPR3, DES, PCDH1, LLGL2, HMGCL, TSPAN, ANXA4, ABP1, ERP70, HSD17B2, FBP1, SIM1, LAF4, CRKL, TOB2, GDA, MMP7, PRSS8, CKS2, PGLYRP, KIF4A, PABPC1, FLJ21865, ETV4, EXTL3, PIK3C2A, DNMT3B, DKFZP566G1424, KIAA0026, CCNB1, GPC3, EVA1, FSTL3, MSLN, FLJ21935, DES, STK17A, KCNH2, DLK1, CPX, NRP1, PCTAIRE2BP, ELF3, AGR2, FLJ10849, ATP1B1, GPX2, SLI, AMPD1, OTC, MMP3, CPX, COX6A2, S100A4, LOC51315, MDS024, PCDH1, PGLYRP, NK4, TRAF2, ARHE, LOC51256, ITGA3, KIAA0971, KIAA1037, GABRA6, U1SNRNPBP, MYBL2, POLA2, TRH, KRTHB6, COL3A1, PDK3, NNAT, FLJ20510, FLJ20208, ANXA4, MMP7, ETS1, ABLIM, CPT1A, BARD1, OKL38, ATP1B1, TONDU, STC1, ABLIM, GCNT3, UCP3, SLC12A2, LOC51141, HSD17B2, ITIH2, INADL, ANXA4, RNASE6PL, CKS2, PABPC1, GJB1, CUTL1, SPTBN1, FLJ13881, LGALS4, C11ORF9, ARSE, LAMB3, MVP, UGT1A1, GLJ20053, CD74, BUB1B, CCND1, CCNE1, TOP2A, TYMS, ALDH1, and CYP3A5). In some embodiments, the effectiveness of the test compound in treating the cancer comprises the ability of the test compound to slow tumor growth. In other embodiments, the effectiveness of said test compound in treating the cancer comprises the ability of the test compound to prevent metastasis of the cancer. In some embodiments, the test compound is a known cancer chemotherapeutic. In other embodiments, the test compound is a candidate cancer therapeutic. In some embodiments, the method further comprises the step of determining the effect of the test compound on the level of expression of one or more drug sensitivity genes in the cancer sample. In some embodiments, detecting the level of expression of one or more drug sensitivity genes comprises detecting the level of mRNA expressed from the drug sensitivity genes. In some embodiments, detecting the level of mRNA expressed from the drug sensitivity genes comprises exposing the mRNA to a nucleic acid probe complementary to the mRNA. In other embodiments, detecting the level of mRNA expressed from the drug sensitivity genes comprises performing an INVADER assay. In some further embodiments, detecting the level of expression of one or more drug sensitivity genes comprises detecting the level of polypeptide expressed from the drug sensitivity genes. In some embodiments, detecting the level of polypeptide expressed from the drug sensitivity genes comprises exposing the polypeptide to an antibody specific to the polypeptide and detecting the binding of the antibody to the polypeptide.

## GENERAL DESCRIPTION OF THE INVENTION

One of the most critical issues to be solved in regard to cancer chemotherapy is the need to establish a method for predicting efficacy or toxicity of anti-cancer drugs for individual patients. To identify genes that are associated with chemosensitivity, methods of the present invention used a cDNA microarray (See *e.g.,* Ross et al., Nat Genet. 24: 227-35 [2000]; Scherf et al., Nat Genet. 24: 236-44 [2000]) representing 23,040 genes to analyze expression profiles in a panel of 85 cancer xenografts derived from nine human organs. The xenografts, implanted into nude mice, were examined for sensitivity to nine anti-cancer drugs (VLB, vinblastine; VCR, vincristine; CPM, cyclophosphamide; 5FU, 5-fluorouracil; MMC, mitomycin; ADR, adriamycin; MTX, methotrexate; ACNU, 3-[(4-amino-2-methyl-5-pyrimidinyl)methyl]-1-(2-chloroethyl)-1-nitrosourea hydrochloride; and DDP, cisplatin). Comparison of the gene-expression profiles of the tumors with sensitivities to each drug identified 1578 genes whose expression levels correlated significantly with chemosensitivity;

333 of those genes showed significant correlation with two or more drugs and 32 correlated with six or seven drugs. These data provide useful information for identifying predictive markers for drug sensitivity that provide selection of "personalized chemotherapy" for individual patients as well as for development of novel drugs to overcome acquired resistance of tumor cells to chemical agents.

To identify molecular markers that predict chemosensitivity of individual tumors, gene-expression profiles of 85 human-cancer xenografts in nude mice were analyzed after treating each mouse with one of nine anti-cancer drugs (Tashiro et al., Cancer Chemother Pharmacol. 24: 187-92 [1989]; Inaba et al., Jpn J Cancer Res. 79: 517-22 [1988]; Maruo et al., Anticancer Res. 10: 209-12 [1990]; Inaba et al., Cancer. 64: 1577-82 [1989]). Although the metabolism and transport of anti-cancer drugs are not exactly the same in both species, in the murine model one can investigate efficacy of a drug at similar concentrations in all the mice examined. Furthermore, repeated and reproducible experiments can be made with xenograft models, an impossibility in clinical cases. For example, one can obtain information about the efficacy of a particular drug by comparing the same xenografted tumors with or without drug treatment. In clinical cases on the other hand, one has to speculate the efficacy of anti-cancer drugs on the basis of changes in tumor size, but that is sometimes very hard to judge because the growth rates of tumors vary significantly from one patient to another. Since accumulated evidence demonstrates that chemosensitivities in xenografts accord well with clinical results (Tashiro et al., Cancer Chemother Pharmacol. 24: 187-92 [1989]; Inaba et al., Jpn J Cancer Res. 79: 517-22 [1988]), to obtain information regarding chemosensitivity vis-a-vis gene expression, an animal model has great advantages.

Experiments conducted during the course of development of the present invention demonstrated that xenografts derived from non-small cell lung cancers and gastric cancers were distributed in multiple branches. As to clinicopathological features, the 11 xenografts derived from non-small cell lung carcinomas consisted of seven large-cell carcinomas, two squamous cell carcinomas (QG56, Lu61) and two papillary adenocarcinomas (LC11, LC17). When a dendrogram was constructed on the basis of gene expression, all xenografts derived from papillary adenocarcinomas and squamous cell carcinomas of the lung were clustered in the same branch, while four of the seven xenografts from large-cell lung carcinomas were clustered in the same branch as non-small cell lung carcinomas and the others were scattered. Likewise, xenografts derived from gastric cancers fell into several different branches, suggesting that the clinico-pathological features of large-cell lung cancer and gastric cancer are relatively heterogeneous (Tahara, Cancer. 75: 1410-7 [1995]; Mackay et al., Ultrastruct Pathol. 13: 561-71 [1989]). Clustering analysis of tumor samples on the basis of gene expression allows for detailed diagnosis of individual cancers. Moreover, one can now recognize a novel type of classification based on gene expression by exploring a large number of cancerous tissues, thus improving current methods of cancer therapy (Alizadeh, Nature 403: 503-11 [2000]).

Although a number of investigators have reported possible regulators related to chemosensitivity of cancer cells (Chen et al., Cell. 47: 381-9 [1986]), the available information is still very limited. The Pearsons correlation analysis undertaken during the development of the present invention identified hundreds of candidate genes associated with sensitivity to anti-cancer drugs in 85 xenografts. Some of the genes listed in Table 2 have also been indicated their relations to chemosensitivity by others, supporting the reliability of the analysis. For example, another study showed that expression levels of topoisomerase II-alpha (*TOP2A*), a molecular target of adriamycin, correlate positively with sensitivity to that drug (Kawanami et al., Oncol Res. 8: 197-206 [1996]; Ramachandran et al., Biochem Pharmacol. 45: 1367-71 [1993]); the data of the present invention also indicated a positive correlation (r=0.44, $p$<0.001) between expression of *TOP2A* and sensitivity to adriamycin among the 85 xenografts. Similarly, abundant expression of thymidylate synthetase (*TYMS*), which inactivates 5-FU to 5-fluoro-dUMP, was reported to correlate with resistance of colon-cancer cells to 5-FU (Johnston et al., Cancer Res. 55: 1407-12 [1995]). The data of the present invention consistently revealed a negative correlation (r=-0.29, $p$<0.01) between expression levels of *TYMS* and sensitivity to 5FU. In addition, aldehyde dehydrogenase 1 *(ALDH1),* which is involved in the resistance of tumor cells to CPM, was negatively correlated with sensitivity to CPM (r=-0.5, $p$<0.001) in accord with a previous report (Chen and Waxman, Biochem Pharmacol. 49: 1691-701 [1995]). Data obtained using the present invention also identified genes that were associated with chemosensitivity in cancers of specific organs (Table 1).

Among the genes showing correlation with two or more anti-cancer drugs, 32 were correlated with sensitivity to either six or seven drugs; such multiple associations are likely to be more meaningful than those of other genes that showed correlation to 1-5 drugs, and these 32 genes or their products should be more useful molecules for diagnosis of chemosensitivity as well as good targets for development of novel drugs to overcome acquired resistance of tumor cells to chemical agents. Of the 32 genes on the list, some have already been shown to be associated with chemosensitivity. For example, major vault protein (*MVP*), a negative regulator for all the drugs tested here except VLB, is essential for normal vault structure. In accord with the data obtained by the present invention, Kitazono *et al.* (Kitazono et al., J Natl Cancer Inst. 91:1647-53 [1999]) demonstrated that *MVP* was involved in resistance to adriamycin, vincristine, VP-16, Taxol, and gramicidine D; this protein appears to have an important role in the transport of adriamycin between nucleus and cytoplasm in the SW-620 human colon carcinoma cell line (Kitazono et al., J Natl Cancer Inst. 91: 1647-53 [1999]; Schroeijers et al., Cancer Res. 60: 1104-10 [2000]; Schroeijers et al., Am J Pathol. 152: 373-8 [1998]). *CYP3A5*, a member of the cytochrome P450 subfamily 3A (heme-thiolate monooxygenases), is involved in an NADPH-dependent electron transport pathway. The product of this gene oxidizes a variety of xenobiotics and is involved in their detoxification (Huang et al., Drug Metab Dispos. 24: 899-905 [1996]; Schuetz et al., Mol Pharmacol. 49: 311-8 [1996]). It also plays a role in the metabolism of CPT-11 (Santos et al., Clin Cancer Res. 6: 2012-20 [2000]). The present invention is not limited to a particular mechanism. Indeed, an understanding of the mechanism of the present invention is not required to practice the present invention. Nonetheless, it is contemplated that increased expression of *CYP3A5* may enhance detoxification and inactivation of anti-cancer drugs and confer drug-resistance on cancer cells. Regarding *UGT1A1,* its significance in the CPT-11 detoxification pathway has been reported (Innocenti et al., Drug Metab Dispos. 29: 596-600 [2001]). The data obtained during the development of the present invention support an important role for *UGT1A1* in the metabolic pathway of other anti-cancer drugs. Galectin 4 (*LGALS4*) is involved in the assembly of adherens junctions and increases malignant potential in some cancers of the digestive system (Kondoh et al., Cancer Res. 59: 4990-6 [1999]; Lotan et al., Carbohydr. Res. 213: 47-57 [1991]). Expression of *LGALS4* is more highly up-regulated in metastatic foci than in corresponding primary tumors (Lotan *et al.,* supra). In addition, genes encoding cell-surface receptors or cell-adhesion molecules, such as *Y7VFRSF14,* laminin, beta 3 (*LAMB3*), gap-junction protein, beta 1 (*GJB1*)*,* and CD74 antigen (*CD74*) are contemplated to be targets for development of novel drugs for overcoming drug resistance. It has been suggested that multidrug resistance proteins are associated with resistance to a wide variety of anti-cancer drugs (Borst et al., J Natl Cancer Inst. 92: 1295-302 [2000]). The cDNA microarray of the present invention includes seven genes belonging to the ABC binding cassette, sub-family B (MDR/TAP). Among them, the expression level of *MDR4* was significantly correlated to the sensitivity to 5FU and VLB ($p<0.01$), that of *MDR3* was correlated to the sensitivity to 5FU ($p<0.01$), and also that of *MDR9* was correlated to the sensitivity to MMC ($p<0.05$).

Experiments conducted during the development of the present invention selected cyclin B1 (*CCNB1*) and budding uninhibited by benzimidazole 1, beta (*BUB1B*) as two genes whose reduced expression is likely to induce chemoresistance in cancer cells. Expression of both genes is cell cycle-dependent and detectable at the G2/M phase (Sugiyama et al., Cancer Res. 59: 4406-12., [1999]; Davenport et al., Genomics. 55: 113-7 [1999]). Since many anti-cancer drugs induce arrest at G2/M (Barlogie and Drewinko, Eur J Cancer. 14: 741-5 [1978]; Rao and Rao, J Natl Cancer Inst. 57: 1139-43 [1976]), it is contemplated that abrogation of cell-cycle arrest due to decreased expression of these two genes may allow cancer cells to survive the stress caused by anti-cancer drugs. The list of genes whose level of expression showed a significant relationship to sensitivity to two or more drugs also includes biologically and medically interesting ones. *GPX2,* which showed significant associations with five drugs, has been reported to protect cancer cells from chlorambucil and melphalan (Leyland-Jones et al., Cancer Res. 51: 587-94 [1991]). Genes associated with DNA repair, *MSH6* (Mut-S homologue 6) and *BARD1* (BRCA1 associated RING domain 1), or those involved in cell-cycle regulation, cyclins D1 (*CCND1*) and E1 *(CCNE1),* have also been associated with response to DNA damage or drug sensitivity (Kornmann et al., Cancer Res. 59: 3505-11 [1999]; Smith and Seo, Anticancer Res. 20: 2537-9 [2000]).

In some embodiments, the present invention provides an algorithm to calculate a "drug sensitivity score" using the value of expression levels of the hundreds of sensitivity related genes to each anti-cancer drug (Table 1). The data presented here provides genes that find use in the diagnosis of chemosensitivity. In some embodiments, drug sensitivity scores are utilized in "personalized medicine," or the use of an appropriate treatment to each individual cancerous case. Clinical investigations further define molecular targets for overcoming drug resistance in tumors. Thus, the present invention provides methods of providing "personalized chemotherapy" with more effective and less harmful anti-cancer drugs, and to improvements in therapeutic effectiveness via the modulation of drugs associated with drug resistance.

**DEFINITIONS**

To facilitate an understanding of the present invention, a number of terms and phrases are defined below:

The term "epitope" as used herein refers to that portion of an antigen that makes contact with a particular antibody.

When a protein or fragment of a protein is used to immunize a host animal, numerous regions of the protein may induce the production of antibodies which bind specifically to a given region or three-dimensional structure on the protein; these regions or structures are referred to as "antigenic determinants". An antigenic determinant may compete with the intact antigen (*i.e.,* the "immunogen" used to elicit the immune response) for binding to an antibody.

The terms "specific binding" or "specifically binding" when used in reference to the interaction of an antibody and a protein or peptide means that the interaction is dependent upon the presence of a particular structure (*i.e.,* the antigenic determinant or epitope) on the protein; in other words the antibody is recognizing and binding to a specific protein structure rather than to proteins in general. For example, if an antibody is specific for epitope "A," the presence of a protein containing epitope A (or free, unlabelled A) in a reaction containing labeled "A" and the antibody will reduce the amount of labeled A bound to the antibody.

As used herein, the terms "non-specific binding" and "background binding" when used in reference to the interaction of an antibody and a protein or peptide refer to an interaction that is not dependent on the presence of a particular structure (*i.e.,* the antibody is binding to proteins in general rather that a particular structure such as an epitope).

As used herein, the term "subject" refers to any animal (*e.g.*, a mammal), including, but not limited to, humans, non-human primates, rodents, and the like, which is to be the recipient of a particular treatment. Typically, the terms "subject" and "patient" are used interchangeably herein in reference to a human subject.

As used herein, the term "characterizing cancer in subject" refers to the identification of one or more properties of a cancer sample in a subject, including but not limited to, the presence of benign, pre-cancerous or cancerous tissue, the stage of the cancer, and the subject's prognosis. Cancers may be characterized by the identification of the expression of one or more cancer marker genes, including but not limited to, the drug sensitivity genes disclosed herein. In some embodiments, characterizing cancer further comprises determining the drug sensitivity profile of the cancer.

As used herein, the term "drug sensitivity profile," for example in reference to the drug sensitivity profile of a cancer or tumor, refers to the sensitivity of the cancer to one or more drugs (*e.g.*, chemotherapy drugs). In preferred embodiments, drug sensitivity profiles comprise drug sensitivity information about multiple drugs commonly used in treatment of the cancer or tumor. In some embodiments, the drug sensitivity profile is determined by measuring the expression levels of specific genes (*e.g.,* "drug sensitivity genes").

As used herein, the term "drug sensitivity gene" refers to a gene whose expression level, alone or in combination with other genes, is correlated with the sensitivity of the cancer to chemotherapeutic agents (*e.g.*, the drug sensitivity profile).

As used herein, the term "a reagent that specifically detects expression levels" refers to reagents used to detect the expression of one or more genes (*e.g.,* including but not limited to, the cancer markers of the present invention). Examples of suitable reagents include but are not limited to, nucleic acid probes capable of specifically hybridizing to the gene of interest, PCR primers capable of specifically amplifying the gene of interest, and antibodies capable of specifically binding to proteins expressed by the gene of interest. Other non-limiting examples can be found in the description and examples below.

As used herein, the term "instructions for using said kit for characterizing cancer in said subject" includes instructions for using the reagents contained in the kit for the characterization of cancer (*e.g.*, the determination of a drug sensitivity profile) in a sample from a subject. In some embodiments, the instructions further comprise the statement of intended use required by the U.S. Food and Drug Administration (FDA) in labeling *in vitro* diagnostic products. The FDA classifies *in vitro* diagnostics as medical devices and requires that they be approved through the 510(k) procedure. Information required in an application under 510(k) includes: 1) The in vitro diagnostic product name, including the trade or proprietary name, the common or usual name, and the classification name of the device; 2) The intended use of the product; 3) The establishment registration number, if applicable, of the owner or operator submitting the 510(k) submission; the class in which the in vitro diagnostic product was placed under section 513 of the FD&C Act, if known, its appropriate panel, or, if the owner or operator determines that the device has not been classified under such section, a statement of that determination and the basis for the determination that the in vitro diagnostic product is not so classified; 4)Proposed labels, labeling and advertisements sufficient to describe the in vitro diagnostic product, its intended use, and directions for use. Where applicable, photographs or engineering drawings should be supplied; 5) A statement indicating that the device is similar to and/or different from other in vitro diagnostic products of comparable type in commercial distribution in the U.S., accompanied by data to support the statement; 6) A 510(k) summary of the safety and effectiveness data upon which the substantial equivalence determination is based; or a statement that the 510(k) safety and effectiveness information supporting the FDA finding of substantial equivalence will be made available to any person within 30 days of a written request; 7) A statement that the submitter believes, to the best of their knowledge, that all data and information submitted in the premarket notification are truthful and accurate and that no material fact has been omitted; 8) Any additional information regarding the in vitro diagnostic product requested that is necessary for the FDA to make a substantial equivalency determination. Additional information is available at the Internet web page of the U.S. FDA.

As used herein, the term "cancer expression profile map" refers to a presentation of expression levels of genes in a particular type of cancer. The map may be presented as a graphical representation (*e.g.,* on paper or on a computer screen), a physical representation (*e.g.*, a gel or array) or a digital representation stored in computer memory. Each map corresponds to a particular type of cancer (*e.g.*, drug resistant or drug sensitive cancers or tumors) and thus provides a template for comparison to a patient sample. In preferred embodiments, maps are generated from pooled samples comprising cancerous samples from a plurality of patients with the same type of cancer.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (*e.g.*, data and instructions) to a computer processor. Examples of computer readable media include, but are not limited to, DVDs, CDs, hard disk drives, magnetic tape and servers for streaming media over networks.

As used herein, the terms "processor" and "central processing unit" or "CPU" are used interchangeably and refer to a device that is able to read a program from a computer memory (*e.g.*, ROM or other computer memory) and perform a set of steps according to the program.

As used herein, the term "providing a prognosis" refers to providing information regarding the impact of the presence of cancer (*e.g.*, as determined by the diagnostic methods of the present invention) on a subject's future health (*e.g.*, expected morbidity or mortality or the responsiveness of the cancer a specific treatment).

As used herein, the term "subject diagnosed with a cancer" refers to a subject who has been tested and found to have cancerous cells. The cancer may be diagnosed using any suitable method, including but not limited to, biopsy, x-ray, blood test, and the diagnostic methods of the present invention.

As used herein, the term "initial diagnosis" refers to results of initial cancer diagnosis (*e.g.* the presence or absence of cancerous cells). An initial diagnosis does not include information about the stage of the cancer or the drug sensitivity profile of the cancer.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylinethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "gene" refers to a nucleic acid (*e.g.,* DNA) sequence that comprises coding sequences necessary for the production of a polypeptide, precursor, or RNA (*e.g.*, rRNA, tRNA). The polypeptide can be encoded by a full length coding sequence or by any portion of the coding sequence so long as the desired activity or functional properties (*e.g.,* enzymatic activity, ligand binding, signal transduction, immunogenicity, etc.) of the full-length or fragment are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with noncoding sequences termed "introns" or "intervening regions"' or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (*e.g.,* mutated, added in multiple copies, linked to non-native regulatory sequences, etc). Heterologous genes are distinguished from endogenous genes in that the heterologous gene sequences are typically joined to DNA sequences that are not found naturally associated with the gene sequences in the chromosome or are associated with portions of the chromosome not found in nature (*e.g.*, genes expressed in loci where the gene is not normally expressed).

As used herein, the term "gene expression" refers to the process of converting genetic information encoded in a gene into RNA (*e.g.*, mRNA, rRNA, tRNA, or snRNA) through "transcription" of the gene (*i.e.*, via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e.,* RNA or protein), while "down-regulation" or "repression" refers to regulation that decrease production. Molecules (*e.g.,* transcription factors) that are involved in up-regulation or down-regulation are often called "activators" and "repressors," respectively.

In addition to containing introns, genomic forms of a gene may also include sequences located on both the 5' and 3' end of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, post-transcriptional cleavage and polyadenylation.

The term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified" or "mutant" refers to a gene or gene product that displays modifications in sequence and or functional properties (*i.e.,* altered characteristics) when compared to the wild-type gene or gene product. It is noted that naturally occurring mutants can be isolated; these are identified by the fact that they have altered characteristics (including altered nucleic acid sequences) when compared to the wild-type gene or gene product.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the terms "an oligonucleotide having a nucleotide sequence encoding a gene" and "polynucleotide having a nucleotide sequence encoding a gene," means a nucleic acid sequence comprising the coding region of a gene or in other words the nucleic acid sequence that encodes a gene product. The coding region may be present in a cDNA, genomic DNA or RNA form. When present in a DNA form, the oligonucleotide or polynucleotide may be single-stranded (i. e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

As used herein, the term "oligonucleotide," refers to a short length of single-stranded polynucleotide chain. Oligonucleotides are typically less than 200 residues long (*e.g.*, between 15 and 100), however, as used herein, the term is also intended to encompass longer polynucleotide chains. Oligonucleotides are often referred to by their length. For example a 24 residue oligonucleotide is referred to as a "24-mer". Oligonucleotides can form secondary and tertiary structures by self-hybridizing or by hybridizing to other polynucleotides. Such structures can include, but are not limited to, duplexes, hairpins, cruciforms, bends, and triplexes.

As used herein, the terms "complementary" or "complementarity" are used in reference to polynucleotides (*i.e.*, a sequence of nucleotides) related by the base-pairing rules. For example, for the sequence "5'-A-G-T-3'" is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e.,* identity). A partially complementary sequence is a nucleic acid molecule that at least partially inhibits a completely complementary nucleic acid molecule from hybridizing to a target nucleic acid is "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.,* the hybridization) of a completely homologous nucleic acid molecule to a target under conditions of low stringency. This is not to say that conditions of low stringency are such that non-specific binding is permitted; low stringency conditions require that the binding of two sequences to one another be a specific (*i.e.,* selective) interaction. The absence of non-specific binding may be tested by the use of a second target that is substantially non-complementary (*e.g.*, less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

A gene may produce multiple RNA species that are generated by differential splicing of the primary RNA transcript, cDNAs that are splice variants of the same gene will contain regions of sequence identity or complete homology (representing the presence of the same exon or portion of the same exon on both cDNAs) and regions of complete non-identity (for example, representing the presence of exon "A" on cDNA 1 wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe that can hybridize (*i.e.,* it is the complement of) the single-stranded nucleic acid sequence under conditions of low stringency as described above.

As used herein, the term "hybridization" is used in reference to the pairing of complementary nucleic acids.

Hybridization and the strength of hybridization (*i.e.,* the strength of the association between the nucleic acids) is impacted by such factors as the degree of complementary between the nucleic acids, stringency of the conditions involved, the $T_m$ of the formed hybrid, and the G:C ratio within the nucleic acids. A single molecule that contains pairing of complementary nucleic acids within its structure is said to be "self-hybridized."

As used herein, the term "$T_m$" is used in reference to the "melting temperature." The melting temperature is the temperature at which a population of double-stranded nucleic acid molecules becomes half dissociated into single strands. The equation for calculating the $T_m$ of nucleic acids is well known in the art. As indicated by standard references, a simple estimate of the $T_m$ value may be calculated by the equation: $T_m = 81.5 + 0.41(\% \text{ G} + \text{C})$, when a nucleic acid is in aqueous solution at 1 M NaCl (See *e.g.,* Anderson and Young, Quantitative Filter Hybridization, in Nucleic Acid Hybridization [1985]). Other references include more sophisticated computations that take structural as well as sequence characteristics into account for the calculation of $T_m$.

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (*e.g.,* sequences with 90% or greater homology), and sequences having only partial homology (*e.g.*, sequences with 50-90% homology). Under 'medium stringency conditions," a nucleic acid sequence of interest will hybridize only to its exact complement, sequences with single base mismatches, and closely relation sequences (*e.g.*, 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

"High stringency conditions" when used in reference to nucleic acid hybridization comprise conditions equivalent to binding or hybridization at 42°C in a solution consisting of 5X SSPE (43.8 g/l NaCl, 6.9 g/l NaH$_2$PO$_4$ H$_2$O and 1.85 g/l EDTA, pH adjusted to 7.4 with NaOH), 0.5% SDS, 5X Denhardt's reagent and 100 $\mu$g/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH$_2$PO$_4$ H$_2$O and 1.85 g/l EDTA, pH adjusted to 7.4 with NaOH), 0.5% SDS, 5X Denhardt's reagent and 100 $\mu$g/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH$_2$PO$_4$ H$_2$O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 $\mu$g/ml denatured salmon sperm DNA followed by washing in a solution comprising 5X SSPE, 0.1% SDS at 42°C when a probe of about 500 nucleotides in length is employed.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.*, the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g.*, increasing the temperature of the hybridization and/or wash steps, the use of formamide in the hybridization solution, etc.) (see definition above for "stringency").

"Amplification" is a special case of nucleic acid replication involving template specificity. It is to be contrasted with non-specific template replication (*i.e.,* replication that is template-dependent but not dependent on a specific template). Template specificity is here distinguished from fidelity of replication (*i.e.*, synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (Kacian et al., Proc. Natl. Acad. Sci. USA 69:3038 [1972]). Other nucleic acids will not be replicated by this amplification enzyme. Similarly, in the case of T7 RNA polymerase, this amplification enzyme has a stringent specificity for its own promoters (Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace, Genomics 4:560 [1989]). Finally, Taq and Pfu polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (H.A. Erlich (ed.), PCR Technology, Stockton Press [1989]).

As used herein, the term "amplifiable nucleic acid" is used in reference to nucleic acids that may be amplified by any amplification method. It is contemplated that "amplifiable nucleic acid" will usually comprise "sample template."

As used herein, the term "sample template" refers to nucleic acid originating from a sample that is analyzed for the presence of "target." In contrast, "background template" is used in reference to nucleic acid other than sample template that may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, that is capable of hybridizing to at least a portion of another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein the term "portion" when in reference to a nucleotide sequence (as in "a portion of a given nucleotide sequence") refers to fragments of that sequence. The fragments may range in size from four nucleotides to the entire nucleotide sequence minus one nucleotide (10 nucleotides, 20, 30, 40, 50, 100, 200, etc.).

As used herein, the term "target," refers to the region of nucleic acid bounded by the primers. Thus, the "target" is sought to be sorted out from other nucleic acid sequences. A "segment" is defined as a region of nucleic acid within the target sequence. As used herein, the term "polymerase chain reaction" ("PCR") refers to the method of K.B. Mullis U.S. Patent Nos. 4,683,195 4,683,202, and 4,965,188, hereby incorporated by reference, which describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of $^{32}$P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process are, themselves, efficient templates for subsequent PCR amplifications. As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

As used herein, the terms "restriction endonucleases" and "restriction enzymes" refer to bacterial enzymes, each of which cut double-stranded DNA at or near a specific nucleotide sequence.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (*e.g.,* a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand (*i.e.,* the oligonucleotide or polynucleotide may be single-stranded), but may contain both the sense and anti-sense strands (*i.e.,* the oligonucleotide or polynucleotide may be double-stranded).

As used herein, the term "purified" or "to purify" refers to the removal of components (*e.g.,* contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

"Amino acid sequence" and terms such as "polypeptide" or "protein" are not meant to limit the amino acid sequence to the complete, native amino acid sequence associated with the recited protein molecule.

The term "native protein" as used herein to indicate that a protein does not contain amino acid residues encoded by vector sequences; that is, the native protein contains only those amino acids found in the protein as it occurs in nature. A native protein may be produced by recombinant means or may be isolated from a naturally occurring source.

As used herein the term "portion" when in reference to a protein (as in "a portion of a given protein") refers to fragments of that protein. The fragments may range in size from four amino acid residues to the entire amino acid sequence minus one amino acid.

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (J. Sambrook et al., Molecular Cloning: A Laboratory Manual, Cold Spring Harbor Press, NY, pp 9.31-9.58 [1989]).

The term "Northern blot," as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (J. Sambrook, *et al., supra,* pp 7.39-7.52 [1989]).

The term "Western blot" refers to the analysis of protein(s) (or polypeptides) immobilized onto a support such as nitrocellulose or a membrane. The proteins are run on acrylamide gels to separate the proteins, followed by transfer of the protein from the gel to a solid support, such as nitrocellulose or a nylon membrane. The immobilized proteins are then exposed to antibodies with reactivity against an antigen of interest. The binding of the antibodies may be detected by various methods, including the use of radiolabeled antibodies.

The term "transgene" as used herein refers to a foreign gene that is placed into an organism by, for example, introducing the foreign gene into newly fertilized eggs or early embryos. The term "foreign gene" refers to any nucleic acid (*e.g.*, gene sequence) that is introduced into the genome of an animal by experimental manipulations and may include gene sequences found in that animal so long as the introduced gene does not reside in the same location as does the naturally occurring gene.

As used, the term "eukaryote" refers to organisms distinguishable from "prokaryotes." It is intended that the term encompass all organisms with cells that exhibit the usual characteristics of eukaryotes, such as the presence of a true nucleus bounded by a nuclear membrane, within which lie the chromosomes, the presence of membrane-bound organelles, and other characteristics commonly observed in eukaryotic organisms. Thus, the term includes, but is not limited to such organisms as fungi, protozoa, and animals (*e.g.*, humans).

As used herein, the term "*in vitro*" refers to an artificial environment and to processes or reactions that occur within an artificial environment. In vitro environments can consist of, but are not limited to, test tubes and cell culture. The term *"in vivo"* refers to the natural environment (*e.g.*, an animal or a cell) and to processes or reaction that occur within a natural environment.

The terms "test compound" and "candidate compound" refer to any chemical entity, pharmaceutical, drug, and the like that is a candidate for use to treat or prevent a disease, illness, sickness, or disorder of bodily function (*e.g.*, cancer). Test compounds comprise both known and potential therapeutic compounds. A test compound can be determined to be therapeutic by screening using the screening methods of the present invention. In some embodiments of the present invention, test compounds include antisense compounds.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the present invention.

## DETAILED DESCRIPTION OF THE INVENTION

The present invention relates to genetic profiles and markers of cancers and provides systems and methods for screening drugs that are effective for specific patients and types of cancers. Certain preferred embodiments are provided below to illustrate features of the present invention.

### I. Identification of Cancer markers

In some embodiments, the present invention provides methods for the identification of cancer markers and the correlation of expression of such cancer markers with drug resistance in cancers.

### A. Identification of Markers

As described below (See Experimental section), experiments conducted during the course of development of the present invention identified a series of cancer markers, or "drug sensitivity genes" whose expression was correlated with the sensitivity of a variety of cancers to nine anti-cancer drugs. When 85 xenografts were analyzed together, a cluster of more than 200 genes appeared to show significant correlation with sensitivity to all nine drugs. 20 genes were identified that correlated with two or more anti-caner drugs. Table 2 summarizes data for 20 genes showing the most significant positive or negative Pearson correlation coefficients to each of the drugs. Seventeen genes appeared to be correlated with all seven drugs, and 15 genes with six. The present invention is not limited to a particular mechanism. Indeed, an understanding of the mechanism is not necessary to practice the present invention. Nonetheless, it is contemplated that the correlation of several genes with sensitivity to multiple drugs suggests that some common mechanisms may be involved in drug response.

The present invention is not limited to a particular drug sensitivity gene. Any suitable gene may be utilized in the diagnostic and therapeutic methods of the present invention, including, but not limited to, ADAMTS2, MSH6, MAGEA6, RPS6KB1, PIK3CB, MDR3, MDR4, MDR9, OS4, KIAA1140, GCNT3, FARP1, PP1044, C11ORF15, KIAA0233, BZRP, PRPS1, HGFAC, TRPC4, LOC56889, FLJ20208, FLJ22833, PGLYRP, TNFRSF14, HLA-B, NPR3, DES, PCDH1, LLGL2, HMGCL, TSPAN, ANXA4, ABP1, ERP70, HSD17B2, FBP1, SIM1, LAF4, CRKL, TOB2, GDA, MMP7, PRSS8, CKS2, PGLYRP, KIF4A, PABPC1, FLJ21865, ETV4, EXTL3, PIK3C2A, DNMT3B, DKFZP566G1424, KIAA0026, CCNB1, GPC3, EVA1, FSTL3, MSLN, FLJ21935, DES, STK17A, KCNH2, DLK1, CPX, NRP1, PCTAIRE2BP, ELF3, AGR2, FUJ10849, ATP1B1, GPX2, SLI, AMPD1, OTC, MMP3, CPX, COX6A2, S100A4, LOC51315, MDS024, PCDH1, PGLYRP, NK4, TRAF2, ARHE, LOC51256, ITGA3, KIAA0971, KIAA1037, GABR.A6, U1SNRNPBP, MYBL2, POLA2,

TRH, KRTHB6, COL3Ai, PDK3, NNAT, FLJ20510, FLJ20208, ANXA4, MMP7, ETS1, ABLIM, CPT1A, BARD1, OKL38, ATP1B1, TONDU, STC1, ABLIM, GCNT3, UCP3, SLC12A2, LOC51141, HSD17B2, ITIH2, INADL, ANXA4, RNASE6PL, CKS2, PABPC1, GJB1, CUTL1, SPTBN1, FLJ13881, LGALS4, C11ORF9, ARSE, LAMB3, MVP, UGT1A1, GLJ20053, CD74, BUB1B, CCND1, CCNE1, TOP2A, TYMS, ALDH1, and CYP3A5. Additional genes may be identified using suitable methods, including, but not limited to, those disclosed herein.

The present invention is not limited to the markers described herein. Any suitable marker that correlates with drug sensitivity may be utilized, including but not limited to, those described in the illustrative examples below. Additional markers are also contemplated to be within the scope of the present invention. Any suitable method may be utilized to identify and characterize cancer markers suitable for use in the methods of the present invention, including but not limited to, those described in the illustrative Examples below. For example, in some embodiments, markers identified as being correlated with drug sensitivity using the gene expression microarray methods of the present invention are further characterized using tissue microarray, immunohistochemistry, Northern blot analysis, siRNA or antisense RNA inhibition, mutation analysis, investigation of expression with clinical outcome, as well as other methods disclosed herein.

The gene expression data generated during experiments conducted during the course of development of the present invention was used to calculate a "drug sensitivity score" for sensitivity related genes. Such drug sensitivity scores find use in a variety of diagnostic and therapeutic applications. Exemplary, non-limiting examples of such applications are described below.

**B. Personalized Medicine**

In some embodiments, the present invention provides compositions and methods for the application of personalized medicine to the treatment of cancer. In preferred embodiments, the presence or expression level of drug sensitivity genes of the present invention is used to provide a drug sensitivity score for a specific cancer. For example, a high drug sensitivity score is indicative of a cancer that is likely to be resistant to common chemotherapy and thus more likely to metastasize. The information provided is also used to direct the course of treatment. For example, if a given cancer is found to be resistant to one of several alternative treatments, a treatment that the cancer is not resistant to is chosen for treatment. In other embodiments, subjects with cancers having a high drug sensitivity score may receive additional therapies (*e.g.,* hormonal or radiation therapies) at an earlier point when they are more likely to be effective (*e.g.*, before metastasis).

The personalized treatment methods of the present invention provide several advantages over traditional methods, which rely on much trial and error, and are generally not customized to the individual patient. Treatment is customized to the gene expression profile of the cancer. This results in improved efficacy of treatment, and in some circumstances, reduced cost of treatment (*e.g.*, only drugs likely to be effective are administered).

In some embodiments, the present invention provides a panel for the analysis of a plurality of markers. The panel allows for the simultaneous analysis of multiple markers correlating with drug sensitivity. For example, a panel may include markers identified as correlating with drug sensitivity or resistance in a given cancer. Depending on the subject, panels may be analyzed alone or in combination in order to provide the best possible diagnosis and prognosis. Markers for inclusion on a panel are selected by screening for their predictive value using any suitable method, including but not limited to, those described in the illustrative examples below.

In other embodiments, the present invention provides an expression profile map comprising expression profiles of cancers of various drug sensitivity scores. Such maps can be used for comparison with patient samples. Any suitable method may be utilized, including but not limited to, by computer comparison of digitized data. The comparison data is used to provide diagnoses and/or prognoses to patients (*e.g.*, personalized treatment).

**C. Detection of Markers**

In some embodiments, the present invention provides methods for detection of expression of cancer markers (*e.g.,* to determine drug sensitivity scores or drug sensitivity profiles). In preferred embodiments, expression is measured directly (*e.g.*, at the RNA or protein level). In some embodiments, expression is detected in tissue samples (*e.g.*, biopsy tissue). In other embodiments, expression is detected in bodily fluids (*e.g.,* including but not limited to, plasma, serum, whole blood, mucus, and urine). The present invention further provides panels and kits for the detection of markers.

**1. Detection of RNA**

In some preferred embodiments, detection of cancer markers (*e.g.,* including but not limited to, those disclosed herein) is detected by measuring the expression of corresponding mRNA in a sample (*e.g.,* cancer tissue), mRNA expression may be measured by any suitable method, including but not limited to, those disclosed below.

In some embodiments, RNA is detected by Northern blot analysis. Northern blot analysis involves the separation of RNA and hybridization of a complementary labeled probe.

In other embodiments, RNA expression is detected by enzymatic cleavage of specific structures (INVADER assay, Third Wave Technologies; *See e.g.,* U.S. Patent Nos. 5,846,717, 6,090,543; 6,001,567; 5,985,557; and 5,994,069; each of which is herein incorporated by reference). The INVADER assay detects specific nucleic acid (*e.g.*, RNA) sequences by using structure-specific enzymes to cleave a complex formed by the hybridization of overlapping oligonucleotide probes.

In still further embodiments, RNA (or corresponding cDNA) is detected by hybridization to a oligonucleotide probe). A variety of hybridization assays using a variety of technologies for hybridization and detection are available. For example, in some embodiments, TaqMan assay (PE Biosystems, Foster City, CA; *See e.g.,* U.S. Patent Nos. 5,962,233 and 5,538,848, each of which is herein incorporated by reference) is utilized. The assay is performed during a PCR reaction. The TaqMan assay exploits the 5'-3' exonuclease activity of the AMPLTTAQ GOLD DNA polymerase. A probe consisting of an oligonucleotide with a 5'-reporter dye (*e.g.*, a fluorescent dye) and a 3'-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In yet other embodiments, reverse-transcriptase PCR (RT-PCR) is used to detect the expression of RNA. In RT-PCR, RNA is enzymatically converted to complementary DNA or "cDNA" using a reverse transcriptase enzyme. The cDNA is then used as a template for a PCR reaction. PCR products can be detected by any suitable method, including but not limited to, gel electrophoresis and staining with a DNA specific stain or hybridization to a labeled probe. In some embodiments, the quantitative reverse transcriptase PCR with standardized mixtures of competitive templates method described in U.S. Patents 5,639,606, 5,643,765, and 5,876,978 (each of which is herein incorporated by reference) is utilized.

## 2. Detection of Protein

In other embodiments, gene expression of cancer markers is detected by measuring the expression of the corresponding protein or polypeptide. Protein expression may be detected by any suitable method. In some embodiments, proteins are detected by immunohistochemistry methods. In other embodiments, proteins are detected by their binding to an antibody raised against the protein. The generation of antibodies is described below.

Antibody binding is detected by techniques known in the art (*e.g.*, radioimmunoassay, BLISA (enzyme-linked immunosorbant assay), "sandwich" immunoassays, immunoradiometric assays, gel diffusion precipitation reactions, immunodiffusion assays, *in situ* immunoassays (*e.g.*, using colloidal gold, enzyme or radioisotope labels, for example), Western blots, precipitation reactions, agglutination assays (*e.g.*, gel agglutination assays, hemagglutination assays, etc.), complement fixation assays, immunofluorescence assays, protein A assays, and immunoelectrophoresis assays, etc.

In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many methods are known in the art for detecting binding in an immunoassay and are within the scope of the present invention.

In some embodiments, an automated detection assay is utilized. Methods for the automation of immunoassays include those described in U.S. Patents 5,885,530, 4,981,785, 6,159,750, and 5,358,691, each of which is herein incorporated by reference. In some embodiments, the analysis and presentation of results is also automated. For example, in some embodiments, software that generates a prognosis based on the presence or absence of a series of proteins corresponding to cancer markers is utilized.

In other embodiments, the immunoassay described in U.S. Patents 5,599,677 and 5,672,480; each of which is herein incorporated by reference.

## 3. Data Analysis

In some embodiments, a computer-based analysis program is used to translate the raw data generated by the detection assay (*e.g.*, the presence, absence, or amount of a given marker or markers) into data of predictive value for a clinician. The clinician can access the predictive data using any suitable means. Thus, in some preferred embodiments, the present invention provides the further benefit that the clinician, who is not likely to be trained in genetics or molecular biology, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (*e.g.*, a biopsy or a serum or urine sample) is obtained from a subject and submitted to a profiling service (*e.g.*, clinical lab at a medical facility, genomic profiling business, etc.), located in any part of the world (*e.g.*, in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g.,* a urine sample) and directly send it to a profiling center. Where the sample comprises previously determined biological information, the information may be directly sent to the profiling service by the subject (*e.g.,* an information card containing the information may be scanned by a computer and the data transmitted to a computer of the profiling center using an electronic communication systems). Once received by the profiling service, the sample is processed and a profile is produced (*i.e.,* expression data), specific for the diagnostic or prognostic information desired for the subject.

The profile data is then prepared in a format suitable for interpretation by a treating clinician. For example, rather than providing raw expression data, the prepared format may represent a diagnosis or risk assessment (*e.g.*, a drug sensitivity score) for the subject, along with recommendations for particular treatment options. The data may be displayed to the clinician by any suitable method. For example, in some embodiments, the profiling service generates a report that can be printed for the clinician (*e.g.,* at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counseling based on the results. In some embodiments, the data is used for research use. For example, the data may be used to further optimize the inclusion or elimination of markers as useful indicators of a particular condition or stage of disease.

### D. Kits

In yet other embodiments, the present invention provides kits for the detection and characterization of cancer. In some embodiments, the kits contain antibodies specific for a cancer marker, in addition to detection reagents and buffers. In other embodiments, the kits contain reagents specific for the detection of mRNA or cDNA (*e.g.*, oligonucleotide probes or primers). In preferred embodiments, the kits contain all of the components necessary to perform a detection assay, including all controls, directions for performing assays, and any necessary software for analysis and presentation of results.

### E. In vivo Imaging

In some embodiments, in vivo imaging techniques are used to visualize the expression of cancer markers in an animal (*e.g.*, a human or non-human mammal). For example, in some embodiments, cancer marker mRNA or protein is labeled using an labeled antibody specific for the cancer marker. A specifically bound and labeled antibody can be detected in an individual using an *in vivo* imaging method, including, but not limited to, radionuclide imaging, positron emission tomography, computerized axial tomography, X-ray or magnetic resonance imaging method, fluorescence detection, and chemiluminescent detection. Methods for generating antibodies to the cancer markers of the present invention are described below.

The in vivo imaging methods of the present invention are useful in the diagnosis of cancers that express the cancer markers of the present invention. In vivo imaging is used to visualize the presence of a marker indicative of the cancer. Such techniques allow for diagnosis without the use of an unpleasant biopsy. The in vivo imaging methods of the present invention are also useful for providing prognoses to cancer patients. For example, the presence of a marker indicative of cancers likely to be drug resistant can be detected. The in vivo imaging methods of the present invention can further be used to detect metastatic cancers in other parts of the body. In still further embodiments, in vivo imaging is use to determine the effect of a candidate therapeutic on expression of a particular gene (*e.g.*, a drug resistance gene).

In some embodiments, reagents (*e.g.,* antibodies) specific for the cancer markers of the present invention are fluorescently labeled. The labeled antibodies are introduced into a subject (*e.g.*, orally or parenterally). Fluorescently labeled antibodies are detected using any suitable method (*e.g.,* using the apparatus described in U.S. Patent 6,198,107, herein incorporated by reference).

In other embodiments, antibodies are radioactively labeled. The use of antibodies for in vivo diagnosis is well known in the art. Sumerdon et al., (Nucl. Med. Biol 17:247-254 [1990] have described an optimized antibody-chelator for the radioimmunoscintographic imaging of tumors using Indium-111 as the label. Griffin et al., (J Clin Onc 9:631-640 [1991]) have described the use of this agent in detecting tumors in patients suspected of having recurrent colorectal cancer. The use of similar agents with paramagnetic ions as labels for magnetic resonance imaging is known in the art (Lauffer, Magnetic Resonance in Medicine 22:339-342 [1991]). The label used will depend on the imaging modality chosen. Radioactive labels such as Indium-111, Technetium-99m, or Iodine-131 can be used for planar scans or single photon emission computed tomography (SPECT). Positron emitting labels such as Fluorine-19 can also be used for positron emission tomography (PET). For MRI, paramagnetic ions such as Gadolinium (III) or Manganese (II) can be used.

Radioactive metals with half-lives ranging from 1 hour to 3.5 days are available for conjugation to antibodies, such as scandium-47 (3.5 days) gallium-67 (2.8 days), gallium-68 (68 minutes), technetiium-99m (6 hours), and indium-111 (3.2 days), of which gallium-67, technetium-99m, and indium-111 are preferable for gamma camera imaging, gallium-68 is preferable for positron emission tomography.

A useful method of labeling antibodies with such radiometals is by means of a bifunctional chelating agent, such as diethylenetriaminepentaacetic acid (DTPA), as described, for example, by Khaw et al. (Science 209:295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int. J. Appl. Radiat. Isot. 33:327 [1982]) for labeling of albumin with In-111, but which can be adapted for labeling of antibodies. A suitable method of labeling antibodies with Tc-99m which does not use chelation with DPTA is the pretinning method of Crockford *et al.,* (U.S. Pat. No. 4,323,546, herein incorporated by reference).

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling antibodies.

In the case of the radiometals conjugated to the specific antibody, it is likewise desirable to introduce as high a proportion of the radiolabel as possible into the antibody molecule without destroying its immunospecificity. A further improvement may be achieved by effecting radiolabeling in the presence of the specific cancer marker of the present invention, to insure that the antigen binding site on the antibody will be protected. The antigen is separated after labeling.

In still further embodiments, *in vivo* biophotonic imaging (Xenogen, Almeda, CA) is utilized for in vivo imaging. This real-time *in vivo* imaging utilizes luciferase. The luciferase gene is incorporated into cells, microorganisms, and animals (*e.g.*, as a fusion protein with a cancer marker of the present invention). When active, it leads to a reaction that emits light. A CCD camera and software is used to capture the image and analyze it.

## II. Antibodies

The present invention provides isolated antibodies. In preferred embodiments, the present invention provides monoclonal antibodies that specifically bind to an isolated polypeptide comprised of at least five amino acid residues of the cancer markers described herein. These antibodies find use in the diagnostic methods described herein.

An antibody against a protein of the present invention may be any monoclonal or polyclonal antibody, as long as it can recognize the protein. Antibodies can be produced by using a protein of the present invention as the antigen according to a conventional antibody or antiserum preparation process.

The present invention contemplates the use of both monoclonal and polyclonal antibodies. Any suitable method may be used to generate the antibodies used in the methods and compositions of the present invention, including but not limited to, those disclosed herein. For example, for preparation of a monoclonal antibody, protein, as such, or together with a suitable carrier or diluent is administered to an animal (*e.g.*, a mammal) under conditions that permit the production of antibodies..For enhancing the antibody production capability, complete or incomplete Freund's adjuvant may be administered. Normally, the protein is administered once every 2 weeks to 6 weeks, in total, about 2 times to about 10 times. Animals suitable for use in such methods include, but are not limited to, primates, rabbits, dogs, guinea pigs, mice, rats, sheep, goats, etc.

For preparing monoclonal antibody-producing cells, an individual animal whose antibody titer has been confirmed (*e.g.*, a mouse) is selected, and 2 days to 5 days after the final immunization, its spleen or lymph node is harvested and antibody-producing cells contained therein are fused with myeloma cells to prepare the desired monoclonal antibody producer hybridoma. Measurement of the antibody titer in antiserum can be carried out, for example, by reacting the labeled protein, as described hereinafter and antiserum and then measuring the activity of the labeling agent bound to the antibody. The cell fusion can be carried out according to known methods, for example, the method described by Koehler and Milstein (Nature 256:495 [1975]). As a fusion promoter, for example, polyethylene glycol (PEG) or Sendai virus (HVJ), preferably PEG is used.

Examples of myeloma cells include NS-1, P3U1, SP2/0, AP-1 and the like. The proportion of the number of antibody producer cells (spleen cells) and the number of myeloma cells to be used is preferably about 1:1 to about 20:

1. PEG (preferably PEG 1000-PEG 6000) is preferably added in concentration of about 10% to about 80%. Cell fusion can be carried out efficiently by incubating a mixture of both cells at about 20°C to about 40°C, preferably about 30°C to about 37°C for about 1 minute to 10 minutes.

Various methods may be used for screening for a hybridoma producing the antibody (*e.g.*, against a tumor antigen or autoantibody of the present invention). For example, where a supernatant of the hybridoma is added to a solid phase (*e.g.*, microplate) to which antibody is adsorbed directly or together with a carrier and then an anti-immunoglobulin antibody (if mouse cells are used in cell fusion, anti-mouse immunoglobulin antibody is used) or Protein A labeled with a radioactive substance or an enzyme is added to detect the monoclonal antibody against the protein bound to the solid phase. Alternately, a supernatant of the hybridoma is added to a solid phase to which an anti-immunoglobulin antibody or Protein A is adsorbed and then the protein labeled with a radioactive substance or an enzyme is added to detect the monoclonal antibody against the protein bound to the solid phase.

Selection of the monoclonal antibody can be carried out according to any known method or its modification. Normally, a medium for animal cells to which HAT (hypoxanthine, aminopterin, thymidine) are added is employed. Any selection and growth medium can be employed as long as the hybridoma can grow. For example, RPMI 1640 medium containing 1% to 20%, preferably 10% to 20% fetal bovine serum, GIT medium containing 1% to 10% fetal bovine serum, a serum free medium for cultivation of a hybridoma (SFM-101, Nissui Seiyaku) and the like can be used. Normally, the cultivation is carried out at 20°C to 40°C, preferably 37°C for about 5 days to 3 weeks, preferably 1 week to 2 weeks under about 5% $CO_2$ gas. The antibody titer of the supernatant of a hybridoma culture can be measured according to the same manner as described above with respect to the antibody titer of the anti-protein in the antiserum.

Separation and purification of a monoclonal antibody (*e.g.*, against a cancer marker of the present invention) can be carried out according to the same manner as those of conventional polyclonal antibodies such as separation and purification of immunoglobulins, for example, salting-out, alcoholic precipitation, isoelectric point precipitation, electrophoresis, adsorption and desorption with ion exchangers (*e.g.*, DEAE), ultracentrifugation, gel filtration, or a specific purification method wherein only an antibody is collected with an active adsorbent such as an antigen-binding solid phase, Protein A or Protein G and dissociating the binding to obtain the antibody.

Polyclonal antibodies may be prepared by any known method or modifications of these methods including obtaining antibodies from patients. For example, a complex of an immunogen (an antigen against the protein) and a carrier protein is prepared and an animal is immunized by the complex according to the same manner as that described with respect to the above monoclonal antibody preparation. A material containing the antibody against is recovered from the immunized animal and the antibody is separated and purified.

As to the complex of the immunogen and the carrier protein to be used for immunization of an animal, any carrier protein and any mixing proportion of the carrier and a hapten can be employed as long as an antibody against the hapten, which is crosslinked on the carrier and used for immunization, is produced efficiently. For example, bovine serum albumin, bovine cycloglobulin, keyhole limpet hemocyanin, etc. may be coupled to an hapten in a weight ratio of about 0.1 part to about 20 parts, preferably, about 1 part to about 5 parts per 1 part of the hapten.

In addition, various condensing agents can be used for coupling of a hapten and a carrier. For example, glutaraldehyde, carbodiimide, maleimide activated ester, activated ester reagents containing thiol group or dithiopyridyl group, and the like find use with the present invention. The condensation product as such or together with a suitable carrier or diluent is administered to a site of an animal that permits the antibody production. For enhancing the antibody production capability, complete or incomplete Freund's adjuvant may be administered. Normally, the protein is administered once every 2 weeks to 6 weeks, in total, about 3 times to about 10 times.

The polyclonal antibody is recovered from blood, ascites and the like, of an animal immunized by the above method. The antibody titer in the antiserum can be measured according to the same manner as that described above with respect to the supernatant of the hybridoma culture. Separation and purification of the antibody can be carried out according to the same separation and purification method of immunoglobulin as that described with respect to the above monoclonal antibody.

The protein used herein as the immunogen is not limited to any particular type of immunogen. For example, a cancer marker of the present invention (further including a gene having a nucleotide sequence partly altered) can be used as the immunogen. Further, fragments of the protein may be used. Fragments may be obtained by any methods including, but not limited to expressing a fragment of the gene, enzymatic processing of the protein, chemical synthesis, and the like.

**III. Drug Screening**

In some embodiments, the present invention provides drug screening assays (*e.g.*, to screen for anticancer drugs). In some embodiments, the effect of therapeutics on the growth and/or progression of cancers with specific drug sensitivity profiles are assessed. Any suitable method for assaying tumor growth or proliferation may be utilized. For example, in some embodiments, the assay described in Example 3 is utilized. The present invention thus provides methods of providing "personalized medicine" for individuals with a given drug sensitivity profile.

In other embodiments, the present invention provides methods of screening for compounds that alter (*e.g.,* increase or decrease) the expression of cancer marker genes. In some embodiments, candidate compounds are antisense agents (*e.g.*, oligonucleotides) directed against cancer markers. See Section IV below for a discussion of antisense therapy. In other embodiments, candidate compounds are antibodies that specifically bind to a cancer marker of the present invention. In still further embodiments, the present invention provides method of screening for compounds that alter (*e.g.*, decrease) the expression of drug resistance genes.

In one screening method, candidate compounds are evaluated for their ability to alter cancer marker expression by contacting a compound with a cell expressing a cancer marker and then assaying for the effect of the candidate compounds on expression. In some embodiments, the effect of candidate compounds on expression of a cancer marker gene is assayed for by detecting the level of cancer marker mRNA expressed by the cell. mRNA expression can be detected by any suitable method. In other embodiments, the effect of candidate compounds on expression of cancer marker genes is assayed by measuring the level of polypeptide encoded by the cancer markers. The level of polypeptide expressed can be measured using any suitable method, including but not limited to, those disclosed herein.

In some embodiments, the present invention provides screening methods for identifying modulators, *i.e.,* candidate or test compounds or agents (*e.g.,* proteins, peptides, peptidomimetics, peptoids, small molecules or other drugs) which bind to cancer markers of the present invention, have an inhibitory (or stimulatory) effect on, for example, cancer marker expression or cancer marker activity, or have a stimulatory or inhibitory effect on, for example, the expression or activity of a cancer marker substrate. Compounds thus identified can be used to modulate the activity of target gene products (*e.g.*, cancer marker genes) either directly or indirectly in a therapeutic protocol, to elaborate the biological function of the target gene product, or to identify compounds that disrupt normal target gene interactions. Compounds which inhibit the activity or expression of cancer markers are useful in the treatment of proliferative disorders, *e.g.*, cancer, particularly metastatic (*e.g.*, androgen independent) prostate cancer.

In one embodiment, the invention provides assays for screening candidate or test compounds that are substrates of a cancer markers protein or polypeptide or a biologically active portion thereof. In another embodiment, the invention provides assays for screening candidate or test compounds that bind to or modulate the activity of a cancer marker protein or polypeptide or a biologically active portion thereof.

The test compounds of the present invention can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g.,* Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckerinann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e.g.,* Houghten, Biotechniques 13:412-421 [1992]), or on beads (Lam, Nature 354:82-84 [1991]), chips (Fodor, Nature 364:555-556 [1993]), bacteria or spores (U.S. Patent No. 5,223,409; herein incorporated by reference), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. Natl. Acad. Sci. 87:6378-6382 [1990]; Felici, J. Mol. Biol. 222:301 [1991]).

In one embodiment, an assay is a cell-based assay in which a cell that expresses a cancer marker protein or biologically active portion thereof is contacted with a test compound, and the ability of the test compound to the modulate cancer marker's activity is determined. Determining the ability of the test compound to modulate cancer marker activity can be accomplished by monitoring, for example, changes in enzymatic activity. The cell, for example, can be of mammalian origin.

The ability of the test compound to modulate cancer marker binding to a compound, *e.g.*, a cancer marker substrate, can also be evaluated. This can be accomplished, for example, by coupling the compound, *e.g.,* the substrate, with a radioisotope or enzymatic label such that binding of the compound, *e.g.*, the substrate, to a cancer marker can be determined by detecting the labeled compound, *e.g.*, substrate, in a complex.

Alternatively, the cancer marker is coupled with a radioisotope or enzymatic label to monitor the ability of a test compound to modulate cancer marker binding to a cancer markers substrate in a complex. For example, compounds (*e.g.*, substrates) can be labeled with $^{125}I$, $^{35}S$ $^{14}C$ or $^{3}H$, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g.*, a cancer marker substrate) to interact with a cancer marker with or without the labeling of any of the interactants can be evaluated. For example, a microphysiometer can be used to detect the interaction of a compound with a cancer marker without the labeling of either the compound or the cancer marker (McConnell et al. Science 257:1906-1912 [1992]). As used herein, a "microphysiometer" (*e.g.*, Cytosensor) is an analytical instrument that measures the rate at which a cell acidifies its environment using a light-addressable potentiometric sensor (LAPS). Changes in this acidification rate can be used as an indicator of the interaction between a compound and cancer markers.

In yet another embodiment, a cell-free assay is provided in which a cancer marker protein or biologically active portion thereof is contacted with a test compound and the ability of the test compound to bind to the cancer marker protein or biologically active portion thereof is evaluated. Preferred biologically active portions of the cancer markers proteins to be used in assays of the present invention include fragments that participate in interactions with substrates or other proteins, *e.g.*, fragments with high surface probability scores.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g.,* using fluorescence energy transfer (FRET) (see, for example, Lakowicz et al., U.S. Patent No. 5,631,169; Stavrianopoulos et al., U.S. Patent No. 4,968,103; each of which is herein incorporated by reference). A fluorophore label is selected such that a first donor molecule's emitted fluorescent energy will be absorbed by a fluorescent label on a second, 'acceptor' molecule, which in turn is able to fluoresce due to the absorbed energy.

Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in 1 5 the assay should be maximal. An FRET binding event can be conveniently measured through standard fluorometric detection means well known in the art (*e.g.*, using a fluorimeter).

In another embodiment, determining the ability of the cancer markers protein to bind to a target molecule can be accomplished using real-time Biomolecular Interaction Analysis (BIA) (see, *e.g.,* Sjolander and Urbaniczky, Anal. Chem. 63:2338-2345 [1991] and Szabo et al. Curr. Opin. Struct. Biol. 5:699-705 [1995]). "Surface plasmon resonance" or "BIA" detects biospecific interactions in real time, without labeling any of the interactants (*e.g.*, BIAcore). Changes in the mass at the binding surface (indicative of a binding event) result in alterations of the refractive index of light near the surface (the optical phenomenon of surface plasmon resonance (SPR)), resulting in a detectable signal that can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize cancer markers, an anti-cancer marker antibody or its target molecule to facilitate separation of complexed from non-complexed forms of one or both of the proteins, as well as to accommodate automation of the assay. Binding of a test compound to a cancer marker protein, or interaction of a cancer marker protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase-cancer marker fusion proteins or glutathione-S-transferase/target fusion proteins can be adsorbed onto glutathione Sepharose beads (Sigma Chemical, St. Louis, MO) or glutathione-derivatized microtiter plates, which are then combined with the test compound or the test compound and either the non-adsorbed target protein or cancer marker protein, and the mixture incubated under conditions conducive for complex formation (*e.g.,* at physiological conditions for salt and pH). Following incubation, the beads or microtiter plate wells are washed to remove any unbound components, the matrix immobilized in the case of beads, complex determined either directly or indirectly, for example, as described above.

Alternatively, the complexes can be dissociated from the matrix, and the level of cancer markers binding or activity determined using standard techniques. Other techniques for immobilizing either cancer markers protein or a target molecule on matrices include using conjugation of biotin and streptavidin. Biotinylated cancer marker protein or target molecules can be prepared from biotin-NHS (N-hydroxy-succinimide) using techniques known in the art (*e.g.*, biotinylation kit, Pierce Chemicals, Rockford, EL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g.*, by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g.*, using a labeled antibody specific for the immobilized component (the antibody, in turn, can be directly labeled or indirectly labeled with, *e.g.*, a labeled anti-IgG antibody).

This assay is performed utilizing antibodies reactive with cancer marker protein or target molecules but which do not interfere with binding of the cancer markers protein to its target molecule. Such antibodies can be derivatized to the wells of the plate, and unbound target or cancer markers protein trapped in the wells by antibody conjugation. Methods for detecting such complexes, in addition to those described above for the GST-immobilized complexes, include immunodetection of complexes using antibodies reactive with the cancer marker protein or target molecule, as well as enzyme-linked assays which rely on detecting an enzymatic activity associated with the cancer marker protein or target molecule.

Alternatively, cell free assays can be conducted in a liquid phase. In such an assay, the reaction products are separated from unreacted components, by any of a number of standard techniques, including, but not limited to: differential centrifugation (see, for example, Rivas and Minton, Trends Biochem Sci 18:284-7 [1993]); chromatography (gel filtration chromatography, ion-exchange chromatography); electrophoresis (*see, e.g.*, Ausubel et al., eds. Current Protocols in Molecular Biology 1999, J. Wiley: New York.); and immunoprecipation (*see*, for example, Ausubel et al., eds. Current Protocols in Molecular Biology 1999, J. Wiley: New York). Such resins and chromatographic techniques are known to one skilled in the art (*See e.g.*, Heegaard J. Mol. Recognit 11:141-8 [1998]; Hageand Tweed J. Chromatogr. Biomed. Sci. Appl 699:499-525 [1997]). Further, fluorescence energy transfer may also be conveniently utilized, as described herein, to detect binding without further purification of the complex from solution.

The assay can include contacting the cancer markers protein or biologically active portion thereof with a known compound that binds the cancer marker to form an assay mixture, contacting the assay mixture with a test compound, and determining the ability of the test compound to interact with a cancer marker protein, wherein determining the ability of the test compound to interact with a cancer marker protein includes determining the ability of the test compound to preferentially bind to cancer markers or biologically active portion thereof, or to modulate the activity of a target molecule, as compared to the known compound.

To the extent that cancer markers can, *in vivo*, interact with one or more cellular or extracellular macromolecules, such as proteins, inhibitors of such an interaction are useful. A homogeneous assay can be used can be used to identify inhibitors.

For example, a preformed complex of the target gene product and the interactive cellular or extracellular binding partner product is prepared such that either the target gene products or their binding partners are labeled, but the signal generated by the label is quenched due to complex formation (see, *e.g.*, U.S. Patent No. 4,109,496, herein incorporated by reference, that utilizes this approach for immunoassays). The addition of a test substance that competes with and displaces one of the species from the preformed complex will result in the generation of a signal above background. In this way, test substances that disrupt target gene product-binding partner interaction can be identified. Alternatively, cancer markers protein can be used as a "bait protein" in a two-hybrid assay or three-hybrid assay (*see, e.g.*, U.S. Patent No. 5,283,317; Zervos et al., Cell 72:223-232 [1993]; Madura et al., J. Biol. Chem. 268.12046-12054 [1993]; Bartel et al., Biotechniques 14:920-924 [1993]; Iwabuchi et al., Oncogene 8:1693-1696 [1993]; and Brent W0 94/10300; each of which is herein incorporated by reference), to identify other proteins, that bind to or interact with cancer markers ("cancer marker-binding proteins" or "cancer marker-bp") and are involved in cancer marker activity. Such cancer marker-bps can be activators or inhibitors of signals by the cancer marker proteins or targets as, for example, downstream elements of a cancer markers-mediated signaling pathway.

Modulators of cancer markers expression can also be identified. For example, a cell or cell free mixture is contacted with a candidate compound and the expression of cancer marker mRNA or protein evaluated relative to the level of expression of cancer marker mRNA or protein in the absence of the candidate compound. When expression of cancer marker mRNA or protein is greater in the presence of the candidate compound than in its absence, the candidate compound is identified as a stimulator of cancer marker mRNA or protein expression. Alternatively, when expression of cancer marker mRNA or protein is less (i.e., statistically significantly less) in the presence of the candidate compound than in its absence, the candidate compound is identified as an inhibitor of cancer marker mRNA or protein expression. The level of cancer markers mRNA or protein expression can be determined by methods described herein for detecting cancer markers mRNA or protein.

A modulating agent can be identified using a cell-based or a cell free assay, and the ability of the agent to modulate the activity of a cancer markers protein can be confirmed *in vivo, e.g.*, in an animal such as an animal model for a disease (*e.g.*, an animal with prostate cancer or metastatic prostate cancer; or an animal harboring a xenograft of a prostate cancer from an animal (*e.g.*, human) or cells from a cancer resulting from metastasis of a prostate cancer (*e.g.*, to a lymph node, bone, or liver), or cells from a prostate cancer cell line.

This invention further pertains to novel agents identified by the above-described screening assays (*See e.g.,* below description of cancer therapies). Accordingly, it is within the scope of this invention to further use an agent identified as described herein (*e.g.,* a cancer marker modulating agent, an antisense cancer marker nucleic acid molecule, a siRNA molecule, a cancer marker specific antibody, or a cancer marker-binding partner) in an appropriate animal model (such as those described herein) to determine the efficacy, toxicity, side effects, or mechanism of action, of treatment with such an agent. Furthermore, novel agents identified by the above-described screening assays can be, *e.g.,* used for treatments as described herein.

**IV. Cancer Therapies**

In some embodiments, the present invention provides therapies for cancer (*e.g.,* personalized treatment identified using the drug screening methods of the present invention). In some embodiments, therapies target cancer markers or drug resistance genes.

**A. Antisense Therapies**

In some embodiments, the present invention targets the expression of cancer markers (*e.g.,* drug sensitivity genes that are overexpressed in drug resistance cancers). For example, in some embodiments, the present invention employs compositions comprising oligomeric antisense compounds, particularly oligonucleotides (*e.g.,* those identified in the drug screening methods described above), for use in modulating the function of nucleic acid molecules encoding cancer markers or drug resistance genes identified using the methods of the present invention. This is accomplished by providing antisense compounds that specifically hybridize with one or more nucleic acids encoding cancer markers or drug resistance genes. The specific hybridization of an oligomeric compound with its target nucleic acid interferes with the normal function of the nucleic acid. This modulation of function of a target nucleic acid by compounds that specifically hybridize to it is generally referred to as "antisense." The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity that may be engaged in or facilitated by the RNA. The overall effect of such interference with target nucleic acid function is modulation of the expression of cancer markers of the present invention. In the context of the present invention, "modulation" means either an increase (stimulation) or a decrease (inhibition) in the expression of a gene. For example, expression may be inhibited to potentially prevent tumor proliferation.

It is preferred to target specific nucleic acids for antisense. "Targeting" an antisense compound to a particular nucleic acid, in the context of the present invention, is a multistep process. The process usually begins with the identification of a nucleic acid sequence whose function is to be modulated. This may be, for example, a cellular gene (or mRNA transcribed from the gene) whose expression is associated with a particular disorder or disease state, or a nucleic acid molecule from an infectious agent. In the present invention, the target is a nucleic acid molecule encoding a cancer marker of the present invention. The targeting process also includes determination of a site or sites within this gene for the antisense interaction to occur such that the desired effect, *e.g.,* detection or modulation of expression of the protein, will result. Within the context of the present invention, a preferred intragenic site is the region encompassing the translation initiation or termination codon of the open reading frame (ORF) of the gene. Since the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the present invention, "start codon" and "translation initiation codon" refer to the codon or codons that are used *in vivo* to initiate translation of an mRNA molecule transcribed from a gene encoding a tumor antigen of the present invention, regardless of the sequence(s) of such codons.

Translation termination codon (or "stop codon") of a gene may have one of three sequences (*i.e.,* 5'-UAA, 5'-UAG and 5'-UGA; the corresponding DNA sequences are 5'-TAA, 5'-TAG and 5'-TGA, respectively). The terms "start codon region" and "translation initiation codon region" refer to a portion of such an mRNA or gene that encompasses from about 25 to about 50 contiguous nucleotides in either direction (*i.e.,* 5' or 3') from a translation initiation codon. Similarly, the terms "stop codon region" and "translation termination codon region" refer to a portion of such an mRNA or gene that encompasses from about 25 to about 50 contiguous nucleotides in either direction (*i.e.,* 5' or 3') from a translation termination codon.

The open reading frame (ORF) or "coding region," which refers to the region between the translation initiation codon and the translation termination codon, is also a region that may be targeted effectively. Other target regions include the 5' untranslated region (5' UTR), referring to the portion of an mRNA in the 5' direction from the translation initiation codon, and thus including nucleotides between the 5' cap site and the translation initiation codon of an mRNA or corresponding nucleotides on the gene, and the 3' untranslated region (3'UTR), referring to the portion of an mRNA in the 3' direction from the translation termination codon, and thus including nucleotides between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The cap region may also be a preferred target region.

Although some eukaryotic mRNA transcripts are directly translated, many contain one or more regions, known as "introns," that are excised from a transcript before it is translated. The remaining (and therefore translated) regions are known as "exons" and are spliced together to form a continuous mRNA sequence. mRNA splice sites (*i.e.,* intron-exon junctions) may also be preferred target regions, and are particularly useful in situations where aberrant splicing is implicated in disease, or where an overproduction of a particular mRNA splice product is implicated in disease. Aberrant fusion junctions due to rearrangements or deletions are also preferred targets. It has also been found that introns can also be effective, and therefore preferred, target regions for antisense compounds targeted, for example, to DNA or pre-mRNA.

In some embodiments, target sites for antisense inhibition are identified using commercially available software programs (*e.g.,* Biognostik, Gottingen, Germany; SysArris Software, Bangalore, India; Antisense Research Group, University of Liverpool, Liverpool, England; GeneTrove, Carlsbad, CA). In other embodiments, target sites for antisense inhibition are identified using the accessible site method described in U.S. Patent WO0198537A2, herein incorporated by reference.

Once one or more target sites have been identified, oligonucleotides are chosen that are sufficiently complementary to the target (*i.e.,* hybridize sufficiently well and with sufficient specificity) to give the desired effect. For example, in preferred embodiments of the present invention, antisense oligonucleotides are targeted to or near the start codon.

In the context of this invention, "hybridization," with respect to antisense compositions and methods, means hydrogen bonding, which may be Watson-Crick, Hoogsteen or reversed Hoogsteen hydrogen bonding, between complementary nucleoside or nucleotide bases. For example, adenine and thymine are complementary nucleobases that pair through the formation of hydrogen bonds. It is understood that the sequence of an antisense compound need not be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired (*i.e.,* under physiological conditions in the case of *in vivo* assays or therapeutic treatment, and in the case of *in vitro* assays, under conditions in which the assays are performed).

Antisense compounds are commonly used as research reagents and diagnostics. For example, antisense oligonucleotides, which are able to inhibit gene expression with specificity, can be used to elucidate the function of particular genes. Antisense compounds are also used, for example, to distinguish between functions of various members of a biological pathway.

The specificity and sensitivity of antisense is also applied for therapeutic uses. For example, antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides are useful therapeutic modalities that can be configured to be useful in treatment regimes for treatment of cells, tissues, and animals, especially humans.

While antisense oligonucleotides are a preferred form of antisense compound, the present invention comprehends other oligomeric antisense compounds, including but not limited to oligonucleotide mimetics such as are described below. The antisense compounds in accordance with this invention preferably comprise from about 8 to about 30 nucleobases (*i.e.,* from about 8 to about 30 linked bases), although both longer and shorter sequences may find use with the present invention. Particularly preferred antisense compounds are antisense oligonucleotides, even more preferably those comprising from about 12 to about 25 nucleobases.

Specific examples of preferred antisense compounds useful with the present invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, modified oligonucleotides that do not have a phosphorus atom in their internucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

In other preferred oligonucleotide mimetics, both the sugar and the internucleoside linkage (*i.e.,* the backbone) of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262, each of which is herein incorporated by reference. Further teaching of PNA compounds can be found in Nielsen *et al.,* Science 254:1497 (1991).

Most preferred embodiments of the invention are oligonucleotides with phosphorothioate backbones and oligonucleosides with heteroatom backbones, and in particular $--CH_2$, $--NH--O--CH_2--$, $--CH_2--N(CH_3)--O--CH_2--$ [known as a methylene (methylimino) or MMI backbone], $--CH_2--O--N(CH_3)--CH_2--$, $--CH_2--N(CH_3)--N(CH_3)--CH_2--$, and $--O--N(CH_3)--CH_2--CH_2--$ [wherein the native phosphodiester backbone is represented as $--O--P--O--CH_2--$] of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred oligonucleotides comprise one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O--$CH_2CH_2OCH_3$, also known as 2'-O-(2-methoxyethyl) or 2'-MOE) (Martin *et al.,* Helv. Chim. Acta 78:486 [1995]) *i.e.,* an alkoxyalkoxy group. A further preferred modification includes 2'-dimethylaminooxyethoxy (*i.e.,* a $O(CH_2)_2ON(CH_3)_2$ group), also known as 2'-DMAOE, and 2'-dimethylaminoethoxyethoxy (also known in the art as 2'-O-dimethylaminoethoxyethyl or 2'-DMAEOE), *i.e.,* 2'-O--$CH_2$--O--$CH_2$--$N(CH_2)_2$.

Other preferred modifications include 2'-methoxy(2'-O--$CH_3$), 2'-aminopropoxy(2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in U.S. Pat. No. 3,687,808. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2. degree °C and are presently preferred base substitutions, even more particularly when combined with 2'-O-methoxyethyl sugar modifications.

Another modification of the oligonucleotides of the present invention involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, (*e.g.*, hexyl-S-tritylthiol), a thiocholesterol, an aliphatic chain, (*e.g.*, dodecandiol or undecyl residues), a phospholipid, (*e.g.,* di-hexadecyl-rac-glycerol or triethylammonium 1,2-di-O-hexadecyl-rac-glycero-3-H-phosphonate), a polyamine or a polyethylene glycol chain or adamantane acetic acid, a palmityl moiety, or an octadecylamine or hexylamino-carbonyl-oxycholesterol moiety.

One skilled in the relevant art knows well how to generate oligonucleotides containing the above-described modifications. The present invention is not limited to the antisensce oligonucleotides described above. Any suitable modification or substitution may be utilized.

It is not necessary for all positions in a given compound to be uniformly modified, and in fact more than one of the aforementioned modifications may be incorporated in a single compound or even at a single nucleoside within an oligonucleotide. The present invention also includes antisense compounds that are chimeric compounds. "Chimeric" antisense compounds or "chimeras," in the context of the present invention, are antisense compounds, particularly oligonucleotides, which contain two or more chemically distinct regions, each made up of at least one monomer unit, *i.e.,* a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNaseH is a cellular endonuclease that cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the present invention may be formed as composite structures of two or more oligonucleotides, modified oligonucleotides, oligonucleosides and/or oligonucleotide mimetics as described above.

The present invention also includes pharmaceutical compositions and formulations that include the antisense compounds of the present invention as described below.

## B. Genetic Therapies

The present invention contemplates the use of any genetic manipulation for use in modulating the expression of cancer markers of the present invention. Examples of genetic manipulation include, but are not limited to, gene knockout (*e.g.,* removing the cancer marker gene from the chromosome using, for example, recombination), expression of antisense constructs with or without inducible promoters, and the like. Delivery of nucleic acid construct to cells *in vitro* or *in vivo* may be conducted using any suitable method. A suitable method is one that introduces the nucleic acid construct into the cell such that the desired event occurs (*e.g.*, expression of an antisense construct).

Introduction of molecules carrying genetic information into cells is achieved by any of various methods including, but not limited to, directed injection of naked DNA constructs, bombardment with gold particles loaded with said constructs, and macromolecule mediated gene transfer using, for example, liposomes, biopolymers, and the like. Preferred methods use gene delivery vehicles derived from viruses, including, but not limited to, adenoviruses, retroviruses, vaccinia viruses, and adeno-associated viruses. Because of the higher efficiency as compared to retroviruses, vectors derived from adenoviruses are the preferred gene delivery vehicles for transferring nucleic acid molecules into host cells *in vivo.* Adenoviral vectors have been shown to provide very efficient *in vivo* gene transfer into a variety of solid tumors in animal models and into human solid tumor xenografts in immune-deficient mice. Examples of adenoviral vectors and methods for gene transfer are described in PCT publications WO 00/12738 and WO 00/09675 and U.S. Pat. Appl. Nos. 6,033,908, 6,019,978, 6,001,557, 5,994,132, 5,994,128, 5,994,106, 5,981,225, 5,885,808, 5,872,154, 5,830,730, and 5,824,544, each of which is herein incorporated by reference in its entirety.

Vectors may be administered to subject in a variety of ways. For example, in some embodiments of the present invention, vectors are administered into tumors or tissue associated with tumors using direct injection. In other embodiments, administration is via the blood or lymphatic circulation (*See e.g.,* PCT publication 99/02685 herein incorporated by reference in its entirety). Exemplary dose levels of adenoviral vector are preferably $10^8$ to $10^{11}$ vector particles added to the perfusate.

## C. Antibody Therapy

In some embodiments, the present invention provides antibodies that target cancers that express a cancer marker or drug resistance protein identified using methods of the present invention. Any suitable antibody (*e.g.*, monoclonal, polyclonal, or synthetic) may be utilized in the therapeutic methods disclosed herein. In preferred embodiments, the antibodies used for cancer therapy are humanized antibodies. Methods for humanizing antibodies are well known in the art (*See e.g.*, U.S. Patents 6,180,370, 5,585,089, 6,054,297, and 5,565,332; each of which is herein incorporated by reference).

In some embodiments, the therapeutic antibodies comprise an antibody generated against a cancer marker or drug resistance protein, wherein the antibody is conjugated to a cytotoxic agent. In such embodiments, a tumor specific therapeutic agent is generated that does not target normal cells, thus reducing many of the detrimental side effects of traditional chemotherapy. For certain applications, it is envisioned that the therapeutic agents will be pharmacologic agents that will serve as useful agents for attachment to antibodies, particularly cytotoxic or otherwise anticellular agents having the ability to kill or suppress the growth or cell division of endothelial cells. The present invention contemplates the use of any pharmacologic agent that can be conjugated to an antibody, and delivered in active form. Exemplary anticellular agents include chemotherapeutic agents, radioisotopes, and cytotoxins. The therapeutic antibodies of the present invention may include a variety of cytotoxic moieties, including but not limited to, radioactive isotopes (*e.g.*, iodine-131, iodine-123, technicium-99m, indium-111, rhenium-188, rhenium-186, gallium-67, copper-67, yttrium-90, iodine-125 or astatine-211), hormones such as a steroid, antimetabolites such as cytosines (*e.g.*, arabinoside, fluorouracil, methotrexate or aminopterin; an anthracycline; mitomycin C), vinca alkaloids (*e.g.*, demecolcine; etoposide; mithramycin), and antitumor alkylating agent such as chlorambucil or melphalan. Other embodiments may include agents such as a coagulant, a cytokine, growth factor, bacterial endotoxin or the lipid A moiety of bacterial endotoxin. For example, in some embodiments, therapeutic agents will include plant-, fungus- or bacteria-derived toxin, such as an A chain toxins, a ribosome inactivating protein, α-sarcin, aspergillin, restrictocin, a ribonuclease, diphtheria toxin or pseudomonas exotoxin, to mention just a few examples. In some preferred embodiments, deglycosylated ricin A chain is utilized.

In any event, it is proposed that agents such as these may, if desired, be successfully conjugated to an antibody, in a manner that will allow their targeting, internalization, release or presentation to blood components at the site of the targeted tumor cells as required using known conjugation technology (See, *e.g.*, Ghose et al., Methods Enzymol., 93: 280 [1983]).

For example, in some embodiments the present invention provides immunotoxins targeted a cancer marker of the present invention (*e.g.*, a drug resistance gene overexpressed in a drug resistant cancer). Immunotoxins are conjugates of a specific targeting agent typically a tumor-directed antibody or fragment, with a cytotoxic agent, such as a toxin moiety. The targeting agent directs the toxin to, and thereby selectively kills, cells carrying the targeted antigen. In some embodiments, therapeutic antibodies employ crosslinkers that provide high *in vivo* stability (Thorpe *et al.*, Cancer Res., 48:6396 [1988]).

In other embodiments, particularly those involving treatment of solid tumors, antibodies are designed to have a cytotoxic or otherwise anticellular effect against the tumor vasculature; by suppressing the growth or cell division of the vascular endothelial cells. This attack is intended to lead to a tumor-localized vascular collapse, depriving the tumor cells, particularly those tumor cells distal of the vasculature, of oxygen and nutrients, ultimately leading to cell death and tumor necrosis.

In preferred embodiments, antibody based therapeutics are formulated as pharmaceutical compositions as described below. In preferred embodiments, administration of an antibody composition of the present invention results in a measurable decrease in cancer (*e.g.*, decrease or elimination of tumor).

## D. Pharmaceutical Compositions

The present invention further provides pharmaceutical compositions (*e.g.*, comprising the antisense or antibody compounds described above). The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary (*e.g.*, by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, *e.g.*, intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

In one embodiment of the present invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

Agents that enhance uptake of oligonucleotides at the cellular level may also be added to the pharmaceutical and other compositions of the present invention. For example, cationic lipids, such as lipofectin (U.S. Pat. No. 5,705,188), cationic glycerol derivatives, and polycationic molecules, such as polylysine (WO 97/30731), also enhance the cellular uptake of oligonucleotides.

The compositions of the present invention may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, *e.g.,* lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Certain embodiments of the invention provide pharmaceutical compositions containing (a) one or more antisense compounds and (b) one or more other chemotherapeutic agents that function by a non-antisense mechanism. Examples of such chemotherapeutic agents include, but are not limited to, anticancer drugs such as daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, vincristine, vinblastine, etoposide, teniposide, cisplatin and diethylstilbestrol (DES). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the invention. Other non-antisense chemotherapeutic agents are also within the scope of this invention. Two or more combined compounds may be used together or sequentially.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates. Optimum dosages may vary depending on the relative potency of individual oligonucleotides, and can generally be estimated based on $EC_{50}$s found to be effective in *in vitro* and *in vivo* animal models or based on the examples described herein. In general, dosage is from 0.01 $\mu$g to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the oligonucleotide is administered in maintenance doses, ranging from 0.01 $\mu$g to 100 g per kg of body weight, once or more daily, to once every 20 years.

## V. Transgenic Animals Expressing Cancer Marker Genes

The present invention contemplates the generation of transgenic animals comprising an exogenous cancer marker gene of the present invention or mutants and variants thereof (*e.g.*, truncations or single nucleotide polymorphisms). In preferred embodiments, the transgenic animal displays an altered phenotype (*e.g.*, increased or decreased drug sensitivity) as compared to wild-type animals. Methods for analyzing the presence or absence of such phenotypes include but are not limited to, those disclosed herein. In some preferred embodiments, the transgenic animals further display an increased or decreased growth of tumors or evidence of cancer.

The transgenic animals of the present invention find use in drug (*e.g.,* cancer therapy) screens. In some embodiments, test compounds (*e.g.*, a drug that is suspected of being useful to treat cancer) and control compounds (*e.g.*, a placebo) are administered to the transgenic animals and the control animals and the effects evaluated.

The transgenic animals can be generated via a variety of methods. In some embodiments, embryonal cells at various developmental stages are used to introduce transgenes for the production of transgenic animals. Different methods are used depending on the stage of development of the embryonal cell. The zygote is the best target for micro-injection. In the mouse, the male pronucleus reaches the size of approximately 20 micrometers in diameter that allows reproducible injection of 1-2 picoliters (pl) of DNA solution. The use of zygotes as a target for gene transfer has a major advantage in that in most cases the injected DNA will be incorporated into the host genome before the first cleavage (Brinster et al., Proc. Natl. Acad. Sci. USA 82:4438-4442 [1985]). As a consequence, all cells of the transgenic non-human animal will carry the incorporated transgene. This will in general also be reflected in the efficient transmission of the transgene to offspring of the founder since 50% of the germ cells will harbor the transgene. U.S. Patent No. 4,873,191 describes a method for the micro-injection of zygotes; the disclosure of this patent is incorporated herein in its entirety.

In other embodiments, retroviral infection is used to introduce transgenes into a non-human animal. In some embodiments, the retroviral vector is utilized to transfect oocytes by injecting the retroviral vector into the perivitelline space of the oocyte (U.S. Pat. No. 6,080,912, incorporated herein by reference). In other embodiments, the developing non-human embryo can be cultured *in vitro* to the blastocyst stage. During this time, the blastomeres can be targets for retroviral infection (Janenich, Proc. Natl. Acad. Sci. USA 73:1260 [1976]). Efficient infection of the blastomeres is obtained by enzymatic treatment to remove the zona pellucida (Hogan et al., in Manipulating the Mouse Embryo, Cold Spring Harbor Laboratory Press, Cold Spring Harbor, N.Y. [1986]). The viral vector system used to introduce the transgene is typically a replication-defective retrovirus carrying the transgene (Jahner et al., Proc. Natl. Acad Sci. USA 82:6927 [1985]). Transfection is easily and efficiently obtained by culturing the blastomeres on a monolayer of virus-producing cells (Stewart, et al., EMBO J., 6:383 [1987]). Alternatively, infection can be performed at a later stage. Virus or virus-producing cells can be injected into the blastocoele (Jahner et al., Nature 298:623 [1982]). Most of the founders will be mosaic for the transgene since incorporation occurs only in a subset of cells that form the transgenic animal. Further, the founder may contain various retroviral insertions of the transgene at different positions in the genome that generally will segregate in the offspring. In addition, it is also possible to introduce transgenes into the germline, albeit with low efficiency, by intrauterine retroviral infection of the midgestation embryo (Jahner *et al., supra* [1982]). Additional means of using retroviruses or retroviral vectors to create transgenic animals known to the art involve the micro-injection of retroviral particles or mitomycin C-treated cells producing retrovirus into the perivitelline space of fertilized eggs or early embryos (PCT International Application WO 90/08832 [1990], and Haskell and Bowen, Mol. Reprod. Dev., 40:386 [1995]).

In other embodiments, the transgene is introduced into embryonic stem cells and the transfected stem cells are utilized to form an embryo. ES cells are obtained by culturing pre-implantation embryos *in vitro* under appropriate conditions (Evans et al., Nature 292:154 [1981]; Bradley et al., Nature 309:255 [1984]; Gossler et al., Proc. Acad. Sci. USA 83:9065 [1986]; and Robertson et al., Nature 322:445 [1986]). Transgenes can be efficiently introduced into the ES cells by DNA transfection by a variety of methods known to the art including calcium phosphate co-precipitation, protoplast or spheroplast fusion, lipofection and DEAE-dextran-mediated transfection. Transgenes may also be introduced into ES cells by retrovirus-mediated transduction or by micro-injection. Such transfected ES cells can thereafter colonize an embryo following their introduction into the blastocoel of a blastocyst-stage embryo and contribute to the germ line of the resulting chimeric animal (for review, See, Jaenisch, Science 240:1468 [1988]). Prior to the introduction of transfected ES cells into the blastocoel, the transfected ES cells may be subjected to various selection protocols to enrich for ES cells which have integrated the transgene assuming that the transgene provides a means for such selection. Alternatively, the polymerase chain reaction may be used to screen for ES cells that have integrated the transgene. This technique obviates the need for growth of the transfected ES cells under appropriate selective conditions prior to transfer into the blastocoel.

In still other embodiments, homologous recombination is utilized to knock-out gene function or create deletion mutants (*e.g.,* truncation mutants). Methods for homologous recombination are described in U.S. Pat. No. 5,614,396, incorporated herein by reference.

**EXPERIMENTAL**

The following examples are provided in order to demonstrate and further illustrate certain preferred embodiments and aspects of the present invention and are not to be construed as limiting the scope thereof.

In the experimental disclosure which follows, the following abbreviations apply: T/C (%), relative tumor volume of the treated mice with respect to the control; VLB, vinblastine; VCR, vincristine; CPM, cyclophosphamide; 5FU, 5-fluorouracil; MMC, mitomycin; ADR, adriamycin; MTX, methotrexate; ACNU, 3-[(4-amino-2-methyl-5-pyrimidinyl)methyl]-1-(2-chloroethyl)-1-nitrosourea hydrochloride; DDP, cisplatin; MTD, maximum tolerated dose.

**EXAMPLE 1**

**Methods**

**Xenografts**

A total of 85 human-cancer xenografts were transplanted to athymic BALB/c-nu/nu mice. The xenografts were maintained by serial subcutaneous transplantation of 2x2x2mm fragments into the right subaxillary region (Clea Japan, Inc., Tokyo) approximately once a month.

**Anti-cancer drugs**

Vinblastine (VLB), Vincristine (VCR) and Cyclophosphamide (CPM) (Shionogi & Co., Osaka); 5-fluorouracil (5FU) (Sigma Chemical Co., St.Louis, MO); Methotrexate (MTX) (Lederle Japan Ltd., Tokyo); Mitomycin-C (MMC) and Adriamycin (ADR) (Kyowa Hakko Kogyo Co., Tokyo); and Nimustine (ACNU) (Sankyo Co., Ltd., Tokyo), were dissolved in sterile 0.85% NaCl just before use. Cisplatin (DDP) (Bristol-Myers Research Institute, Ltd., Tokyo) was dissolved in sterile 0.85% NaCl containing 1% mannitol. The maximum tolerated dose (MTD) for each of these drugs was determined as described previously (Inaba et al., Jpn J Cancer Res. 79:517 [1988]).

**Examination of xenografts for chemosensitivity**

Each anti-cancer drug was administered individually, at the maximum tolerated dose (MTD), to nude mice bearing human-cancer xenografts. Treatment with a given drug commenced when the tumor volume reached 100-300mm$^3$. Drugs were administered by daily intravenous injection for five days in the case of 5-FU and MTX, or as a single administration for all other drugs. The average relative tumor volume (T/C (%)) of six treated mice for each drug with respect to six untreated controls for each xenograft was measured on any given experimental day. Chemosensitivity was represented by a T/C (%) value on day 14 (Ohnishi, Y. Anticancer Drug Sensitivities of Human Tumors Transplanted in Nude Mice. In: Nomura, T., Sakurai, Y., Inaba, M., The Nude Mouse and Anticancer Drug Evaluation, pp. 219-223. Kanagawa: Central Institute of Experimental Animals, 1996).

**Extraction and purification of RNA and preparation of probes**

Total RNA was extracted from each xenograft using TRIZOL reagent (GIBCO) and purified using an mRNA Purification Kit (Amersham Pharmacia Biotech) according to the supplier's protocols. PolyA RNAs from xenografts, and a mixture of pooled mRNA from four normal human tissues (brain, lung, liver, and kidney) purchased from Clontech, were amplified using Ampliscribe T7 Transcription Kits (Epicentre Technologies) and labeled with Cy5-dCTP and Cy3-dCTP respectively, as described previously (Ono et al., Cancer Res. 60:5007 [2000]).

**cDNA microarrays**

A "genome-wide" cDNA microarray system containing 23,040 cDNAs selected from the UniGene database of the National Center for Biotechnology Information was established. Fabrication of the microarray, hybridization, washing, and detection of signal intensities were described previously (Ono et al., Cancer Res. 60:5007 [2000]). To normalize the amount of mRNA between tumors and controls, the Cy5/Cy3 ratio for each gene's expression was adjusted so that the averaged Cy5/Cy3 ratio of 52 housekeeping genes was 1.0. (Ono *et al.,* supra). A cut-off value was assigned, using a variance analysis, to each microarray slide. Genes whose Cy3- or Cy5- signal intensities were lower than the cut-off values were excluded from further investigation. Data from genes where the signal/noise ratio was less than 3 was also excluded.

**Cluster analysis of expression profiles**

Hierarchical clustering analysis for both genes and tumor samples was carried out using web-available software ("Cluster" and "TreeView") written by M. Eisen (available at the Eisen laboratory web site at Lawrence Berkeley National Lab). To obtain reproducible clusters only genes that passed a filter protocol designed to exclude 1) genes where both Cy3 and Cy5 signal intensities were lower than the cut-off value; 2) genes where values were obtained in less than 50% of the samples tested; or 3) genes with standard deviations in observed values of less than 2.0, were selected. Before the clustering algorithm was applied, the fluorescence ratio for each spot was first log-transformed and then the data for each sample were centered to remove experimental biases.

**Identification of genes associated with anti-cancer drugs**

To estimate correlation between the expression ratio ($\log_2$ Cy5/Cy3) and sensitivity to each drug, a Pearson correlation coefficient was calculated by the following formula:

$$r = \mathrm{sigma}(x_i\text{-}x_{mean})(y_i\text{-}y_{mean}) \,/\, \mathrm{root}\,(\mathrm{sigma}\,(x_i\text{-}x_{mean})^2(y_i\text{-}y_{mean})^2)$$

where xi represented the log expression ratio ($\log_2$ Cy5/Cy3) of gene x in xenograft i, while yi represented sensitivity (1-T/C) of xenograft i to drug y. $x_{mean}$ represented the mean of the log expression ratio of gene x, and $y_{mean}$ represented the mean sensitivity (1-T/C) of the drug. Genes were selected showing significant correlation ($p$<0.01) and the absolute value of the slope of the regression line greater than 1.5, when the difference of the T/C values between the most and the least sensitive samples was fixed as one.

**Drug sensitivity score**

Using values of relative expression ratio of the genes associated with anti-cancer drug (Table 1), the algorithm to calculate "drug sensitivity score" was established. The sum of $\log_2$ (Cy5/Cy3) of genes multiplied by the value of Pearsons correlation coefficients for each 85 xenografts was calculated as described previously (Kihara *et al.,* Cancer Res. 61:6474 [2001]).

**Example 2**

**Cluster analysis of gene-expression profiles of 85 xenografts**

The expression profiles of 85 xenografts were subjected to a hierarchical clustering analysis, to investigate similarities among them. Reproducible clusters were obtained with 961 genes; their expression patterns across the 85 xenografts were displayed. The expression profiles of xenograft MC9, analyzed in duplicate independently, were clustered into the closest branch among the xenografts in the sample axis. Xenografts derived from glioblastoma, neuroblastoma, small-cell lung carcinoma, and choriocarcinoma were correctly categorized into single tissues-specific branches. However, xenografts established from carcinomas of the breast, colon, pancreas, stomach, and ovary, as well as non-small cell lung carcinomas, were not clustered into single branches, suggesting that those tumors had heterogeneous expression profiles that reflected wider differences in their histological and/or biological natures.

**Example 3**

**Identification of genes associated with chemosensitivity**

To identify genes having significant associations with efficacy of one or more of the nine anti-cancer drugs (5FU, ACNU, ADR, CPM, DDP, MMC, MTX, VCR, and VLB) examined in the nude-mice system, expression profiles of the genes filtered according to criteria described in Example 1 were analyzed. Pearson correlation coefficients between the expression level of each filtered gene and chemosensitivity to each drug across the 85 cancer xenografts were calculated. As shown in Table 1, when 85 xenografts were analyzed together, a cluster of more than 200 genes appeared to show significant correlation with sensitivity to all nine drugs.

**TABLE 1**

The number of genes that have significant correlation with the sensitivity to nine anti-cancer drugs.

| | All Xenografts (85) | NSCLC (11) | Breast Cancer (14) | Gastric Cancer (13) |
|---|---|---|---|---|
| 5FU | 240 | 184 | 69 | 72 |
| ACNU | 290 | 151 | 78 | 101 |
| ADR | 283 | 72 | 94 | 93 |
| CPM | 459 | 168 | 103 | 92 |
| DDP | 217 | 166 | 76 | 100 |
| MMC | 321 | 137 | 199 | 102 |
| MTX | 219 | 92 | 75 | 171 |
| VCR | 244 | 200 | 107 | 112 |
| VLB | 210 | 201 | 109 | 60 |

These genes were identified not only in 85 xenografts but also in xenografts from three kinds of organs in order to explore the genes related to tissue-dependent chemosensitivity. These genes were satisfied following criteria; P value was smaller than 0.01and the absolute value of the slope of regression line is larger than 1.5 (see materials and methods).

Expression levels of 1578 genes were associated with sensitivity to at least one of the drugs examined. When the data were separately analyzed on the basis of the specific organs from which the xenografts had originated, the statistical power to identify significant associations was lower because the data that discriminated between sensitive and resistant phenotypes were limited. However, since xenografts of non-small cell lung cancer, breast cancer and gastric cancer showed more variable responses to the panel of drugs than xenografts derived from other organs, significant association data for xenografts derived from those three tissues was still obtained. Even though the number of genes selected was smaller, more significant correlation of expression levels of some genes with chemosensitivity in these three tissues than the correlation obtained by analysis using all 85 xenografts together was found. For example, among the 1578 genes mentioned above, the correlation value between the expression level of glutathione peroxidase 2 (*GPX2*) and sensitivity to CPM in the 11 xenografts from non-small cell lung cancer (r=-0.94) was more significant than that of all 85 xenografts (r=-0.59). Similarly, mitogen-activated protein kinase kinase 3 (*MAP2K3*) revealed a stronger correlation between expression level and sensitivity to VCR in the 14 xenografts from breast cancer (r=-0.91, $p<0.001$) than in all xenografts combined (r=-0.29, $p<0.01$). Therefore these two genes seem to be closely involved in tissue-specific efficacy of CPM or VCR, especially as regards non-small cell lung cancers or breast cancers.

**Example 4**

Identification of genes correlated with two or more anti-cancer drugs

Table 2 summarizes data for 20 genes showing the most significant positive or negative Pearson correlation coefficients to each of the drugs, and corresponding p values calculated on the basis of expression profile data from all 85 xenografts. r; Pearson correlation coefficient, slope; the slope of regression line.

**TABLE 2**

| Drug | Unigene | r | slope | P value | Symbol | Description |
|---|---|---|---|---|---|---|
| 5FU | Hs.104935 | -0.84 | -7.4 | 0.001 | | ESTs |
| | Hs.120016 | 0.82 | 3.41 | 0.001 | | EST |
| | Hs.120330 | 0.63 | 3.35 | 0.001 | ADAMTS2 | a disintegrin-like and metalloprotease (reprolysin type) with thrombospondin type 1 motif, 2 |
| | Hs.278460 | 0.6 | 6.09 | 0.001 | MAGEA6 | melanoma antigen, family A, 6 |
| | Hs.86858 | -0.57 | -2.72 | 0.001 | RPS6KB1 | ribosomal protein S6 kinase, 70kD, polypeptide 1 |
| | Hs.239818 | 0.55 | 2.67 | 0.001 | PIK3CB | phosphoinositide-3-kinase, catalytic, beta polypeptide |
| | Hs.180669 | 0.54 | 2.46 | 0.001 | OS4 | conserved gene amplified in osteosarcoma |
| | Hs.131728 | 0.53 | 2.26 | 0.001 | KIAA1140 | KIAA1140 protein |

(continued)

| Drug | Unigene | r | slope | P value | Symbol | Description |
|---|---|---|---|---|---|---|
| | Hs.194710 | 0.52 | 6.82 | 0.001 | GCNT3 | glucosaminyl (N-acetyl) transferase 3, mucin type |
| | Hs.183738 | -0.48 | -2.54 | 0.001 | FARP1 | FERM, RhoGEF (ARHGEF) and pleckstrin domain protein 1 (chondrocyte-derived) |
| | Hs.7212 | -0.47 | -4.61 | 0.001 | PP1044 | hypothetical protein PP1044 |
| | Hs.121619 | 0.47 | 2.01 | 0.001 | C110RF15 | chromosome 11 open reading frame 15 |
| | Hs.88252 | -0.46 | -1.89 | 0.001 | | ESTs |
| | Hs.79077 | 0.44 | 3.06 | 0.001 | KIAA0233 | KIAA0233 gene product |
| | Hs.202 | 0.44 | 2.72 | 0.001 | BZRP | benzodiazapine receptor (peripheral) |
| | Hs.80285 | -0.44 | -1.58 | 0.001 | | Homo sapiens mRNA; cDNA DKFZp586C1723 (from clone DKFZp586C1723) |
| | Hs.56 | -0.42 | -2.53 | 0.001 | PRPS1 | phosphoribosyl pyrophosphate synthetase 1 |
| | Hs.12840 | -0.42 | -1.59 | 0.001 | | Homo sapiens germline mRNA sequence |
| | Hs.104 | -0.42 | -1.81 | 0.001 | HGFAC | HGF activator |
| | Hs.49434 | 0.41 | 2.35 | 0.001 | | ESTs |
| CDDP | Hs.42635 | -0.84 | -1.7 | 0.001 | | ESTs |
| | Hs.262960 | -0.8 | -1.62 | 0.001 | TRPC4 | transient receptor potential channel 4 |
| | Hs.8203 | -0.7 | -2.21 | 0.001 | LOC56889 | endomembrane protein emp70 precursor isolog |
| | Hs.131776 | 0.67 | 2.37 | 0.001 | FLJ20208 | hypothetical protein FLJ20208 |
| | Hs.118183 | 0.62 | 3.9 | 0.001 | FLJ22833 | hypothetical protein FLJ22833 |
| | Hs.137583 | -0.59 | -2.29 | 0.001 | PGLYRP | peptidoglycan recognition protein |
| | Hs.279899 | -0.58 | -2.92 | 0.001 | TNFRSF14 | tumor necrosis factor receptor superfamily, member 14 (herpesvirus entry mediator) |
| | Hs.13234 | -0.58 | -3.52 | 0.001 | | ESTs |
| | Hs.77961 | -0.57 | -2.59 | 0.001 | HLA-B | major histocompatibility complex, class I, B |
| | Hs.123655 | -0.56 | -2.17 | 0.001 | NPR3 | natriuretic peptide receptor C/ guanylate cyclase C (atrionatriuretic peptide receptor C) |
| | Hs.279604 | 0.54 | 2.32 | 0.001 | DES | desmin |
| | Hs.79769 | -0.52 | -2.82 | 0.001 | PCDH1 | protocadherin 1 (cadherin-like 1) |
| | Hs.3123 | -0.51 | -2.42 | 0.001 | LLGL2 | lethal giant larvae (Drosophila) homolog 2 |
| | Hs.831 | -0.51 | -1.85 | 0.001 | HMGCL | 3-hydroxymethyl-3-methylglutaryl-Coenzyme A lyase (hydroxymethylglutaricaciduria) |
| | Hs.7353 | 0.51 | 1.83 | 0.001 | | ESTs, Weakly similar to dJ889M15.3 [H.sapiens] |
| | Hs.38972 | -0.5 | -4.33 | 0.001 | TSPAN | tetraspan 1 |
| | Hs.77840 | -0.49 | -3.41 | 0.001 | ANXA4 | annexin A4 |
| | Hs.75741 | -0.48 | -3.09 | 0.001 | ABP1 | amiloride binding protein 1 (amine oxidase (copper-containing)) |
| | Hs.155109 | -0.47 | -2.99 | 0.001 | HSD17B2 | hydroxysteroid (17-beta) dehydrogenase 2 |
| | Hs.574 | -0.47 | -2.61 | 0.001 | FBP1 | fructose,6-bisphosphatase 1 |

(continued)

| Drug | Unigene | r | slope | P value | Symbol | Description |
|---|---|---|---|---|---|---|
| ACNU | | | | | | |
| | Hs.37902 | -0.98 | -1.99 | 0.001 | | ESTs |
| | Hs.7473 | -0.93 | -6.12 | 0.001 | | ESTs |
| | Hs.66087 | 0.92 | 3.12 | 0.001 | | ESTs |
| | Hs.131301 | -0.89 | -1.61 | 0.001 | | ESTs |
| | Hs.105925 | -0.87 | -2.13 | 0.001 | SIM1 | single-minded (Drosophila) homolog 1 |
| | Hs.38070 | -0.85 | -2.21 | 0.001 | LAF4 | lymphoid nuclear protein related to AF4 |
| | Hs.7033 | 0.85 | 1.58 | 0.001 | | ESTs |
| | Hs.231500 | 0.75 | 2.66 | 0.001 | | EST |
| | Hs.37078 | -0.73 | -1.7 | 0.001 | CRKL | v-crk avian sarcoma virus CT10 oncogene homolog-like |
| | Hs.4994 | -0.73 | -2.06 | 0.001 | TOB2 | transducer of ERBB2, 2 |
| | Hs.239147 | -0.62 | -2.21 | 0.001 | GDA | guanine deaminase |
| | Hs.2256 | -0.61 | -4.62 | 0.001 | MMP7 | matrix metalloproteinase 7 (matrilysin, uterine) |
| | Hs.75799 | -0.59 | -3.58 | 0.001 | PRSS8 | protease, serine, 8 (prostasin) |
| | Hs.83758 | 0.58 | 2.43 | 0.001 | CKS2 | CDC28 protein kinase 2 |
| | Hs.137583 | -0.58 | -2.02 | 0.001 | PGLYRP | peptidoglycan recognition protein |
| | Hs.279766 | 0.56 | 2.16 | 0.001 | KIF4A | kinesin family member 4A |
| | Hs.172182 | -0.54 | -1.83 | 0.001 | PABPC1 | poly(A)-binding protein, cytoplasmic 1 |
| | Hs.29288 | -0.54 | -1.83 | 0.001 | FLJ21865 | hypothetical protein FLJ21865 |
| | Hs.77711 | -0.53 | -2.5 | 0.001 | ETV4 | ets variant gene 4 (E1A enhancer-binding protein, E1AF) |
| | Hs.9018 | 0.53 | 2.23 | 0.001 | EXTL3 | exostoses (multiple)-like 3 |
| ADR | | | | | | |
| | Hs.249235 | 0.9 | 2.33 | 0.001 | PIK3C2A | phosphoinositide-3-kinase, class 2, alpha polypeptide |
| | Hs.122874 | -0.82 | -1.72 | 0.001 | | EST |
| | Hs.131315 | -0.8 | -3.08 | 0.001 | | EST |
| | | | | | | ESTs, Weakly similar to Wiskott-Aldrich |
| | Hs.172330 | 0.79 | 4.18 | 0.001 | | Syndrome protein [H.sapiens] |
| | Hs.35586 | 0.74 | 2.69 | 0.001 | | ESTs |
| | Hs.105119 | 0.68 | 1.66 | 0.001 | | ESTs |
| | Hs.239706 | 0.67 | 2.3 | 0.001 | | Homo sapiens cDNA FLJ12080 fis, clone HEMBB1002477, highly similar to Human Grb2-associated binder mRNA |
| | Hs.55560 | 0.67 | 3.83 | 0.001 | | ESTs |
| | Hs.251673 | 0.63 | 2.98 | 0.001 | DNMT3B | DNA (cytosine-5-)-methyltransferase 3 beta |
| | Hs.19221 | -0.61 | -1.99 | 0.001 | DKFZP566G1424 | hypothetical protein DKFZp566G1424 |
| | Hs.22011 | 0.6 | 3.52 | 0.001 | | ESTs |
| | Hs.71968 | 0.57 | 1.75 | 0.001 | | Homo sapiens mRNA; cDNA DKFZp564F053 (from clone DKFZp564F053) |
| | Hs.173714 | 0.54 | 1.6 | 0.001 | KIAA0026 | MORF-related gene X |
| | Hs.23960 | 0.52 | 2.69 | 0.001 | CCNB1 | cyclin B1 |
| | Hs.119651 | 0.52 | 3.68 | 0.001 | GPC3 | glypican 3 |
| | Hs.116651 | -0.5 | -2.2 | 0.001 | EVA1 | epithelial V-like antigen 1 |

(continued)

| Drug | Unigene | r | slope | P value | Symbol | Description |
|---|---|---|---|---|---|---|
| | Hs.25348 | 0.5 | 3.35 | 0.001 | FSTL3 | follistatin-like 3 (secreted glycoprotein) |
| | Hs.155981 | -0.49 | -4.22 | 0.001 | MSLN | mesothelin |
| | Hs.55016 | 0.49 | 2.98 | 0.001 | FLJ21935 | hypothetical protein FLJ21935 |
| | Hs.279604 | 0.49 | 2.32 | 0.001 | DES | desmin |
| CPM | | | | | | |
| | Hs.34720 | 0.99 | 1.9 | 0.001 | | ESTs |
| | Hs.126501 | 0.98 | 1.83 | 0.001 | | ESTs |
| | Hs.9075 | 0.93 | 2.78 | 0.001 | STK17A | serine/threonine kinase 17a (apoptosis-inducing) |
| | Hs.49076 | -0.88 | -4.79 | 0.001 | | ESTs |
| | Hs.130626 | -0.85 | -3.14 | 0.001 | | ESTs |
| | Hs.188021 | 0.78 | 3.72 | 0.001 | KCNH2 | potassium voltage-gated channel, subfamily H (eag-related), member 2 |
| | Hs.169228 | 0.78 | 7.35 | 0.001 | DLK1 | delta-like homolog (Drosophila) |
| | Hs.301396 | -0.7 | -1.5 | 0.001 | | Homo sapiens cDNA FLJ12625 fis, clone NT2RM4001783, weakly similar to ZINC FINGER PROTEIN HRX |
| | Hs.28164 | 0.67 | 4.15 | 0.001 | | ESTs |
| | Hs.177536 | 0.67 | 3.02 | 0.001 | CPX | metallocarboxypeptidase CPX |
| | Hs.69285 | 0.67 | 3.13 | 0.001 | NRP1 | neuropilin 1 |
| | Hs.283761 | -0.64 | -2.2 | 0.001 | PCTAIRE2BP | tudor repeat associator with PCTAIRE 2 |
| | Hs.184319 | -0.64 | -1.93 | 0.001 | | ESTs, Weakly similar to KIAA1006 protein [H.sapiens] |
| | Hs.166096 | -0.62 | -3.83 | 0.001 | ELF3 | E74-like factor 3 (ets domain transcription factor, epithelial-specific) |
| | Hs.91011 | -0.62 | -5.97 | 0.001 | AGR2 | anterior gradient 2 (Xenepus laevis) homolog |
| | Hs.172753 | 0.6 | 1.85 | 0.001 | | ESTs |
| | Hs.8768 | 0.6 | 1.81 | 0.001 | FLJ10849 | hypothetical protein FLJ10849 |
| | Hs.78629 | -0.59 | -3.49 | 0.001 | ATP1B1 | ATPase, Na+/K+ transporting, beta 1 polypeptide |
| | Hs.2704 | -0.59 | -5.44 | 0.001 | GPX2 | glutathione peroxidase 2 (gastrointestinal) |
| | Hs.3496 | -0.59 | -1.67 | 0.001 | | ESTs |
| MMC | | | | | | |
| | Hs.30965 | -0.97 | -4.3 | 0.001 | SLI | neuronal Shc adaptor homolog |
| | Hs.89570 | -0.97 | -11.03 | 0.001 | AMPD1 | adenosine monophosphate deaminase 1 (isoform M) |
| | Hs.129472 | -0.95 | -4.42 | 0.001 | | ESTs |
| | Hs.117050 | -0.95 | -2.34 | 0.001 | OTC | omithine carbamoyltransferase |
| | Hs.131301 | -0.94 | -2.85 | 0.001 | | ESTs |
| | Hs.83326 | -0.7 | -4.74 | 0.001 | MMP3 | matrix metalloproteinase 3 (stromelysin 1, progelatinase) |
| | Hs.172148 | -0.68 | -2.69 | 0.001 | | ESTs |
| | Hs.177536 | 0.67 | 5.25 | 0.001 | CPX | metallocarboxypeptidase CPX |
| | Hs.250760 | -0.65 | -2.27 | 0.001 | COX6A2 | cytochrome c oxidase subunit Via polypeptide 2 |

(continued)

| Drug | Unigene | r | slope | P value | Symbol | Description |
|---|---|---|---|---|---|---|
| | Hs.81256 | -0.57 | -2.2 | 0.001 | S100A4 | S100 calcium-binding protein A4 (calcium protein, calvasculin, metastasin, murine placental homolog) |
| | Hs.5721 | -0.56 | -1.69 | 0.001 | LOC51315 | hypothetical protein |
| | Hs.112877 | -0.53 | -1.58 | 0.001 | | ESTs |
| | Hs.286122 | 0.52 | 1.57 | 0.001 | MDS024 | MDS024 protein |
| | Hs.79769 | -0.5 | -3.93 | 0.001 | PCDH1 | protocadherin 1 (cadherin-like 1) |
| | Hs.137583 | -0.49 | -2.61 | 0.001 | PGLYRP | peptidoglycan recognition protein |
| | Hs.943 | -0.48 | -3.23 | 0.001 | NK4 | natural killer cell transcript 4 |
| | Hs.200526 | -0.48 | -2.12 | 0.001 | TRAF2 | TNF receptor-associated factor 2 |
| | Hs.279899 | -0.47 | -3.36 | 0.001 | TNFRSF14 | tumor necrosis factor receptor superfamily, member 14 (herpesvirus entry mediator) |
| | Hs.6838 | -0.47 | -2.32 | 0.001 | ARHE | ras homolog gene family, member E |
| | Hs.8645 | 0.47 | 2.83 | 0.001 | LOC51256 | hypothetical protein |
| | Hs.236443 | -0.46 | -2.23 | 0.001 | | Homo sapiens mRNA; cDNA DKFZp564N1063 (from clone DKFZp564N1063) |
| | Hs.265829 | -0.46 | -4.29 | 0.001 | ITGA3 | integrin, alpha 3 (antigen CD49C, alpha 3 subunit of VLA-3 receptor) |
| MTX | | | | | | |
| | Hs.84429 | -0.74 | -3.27 | 0.001 | KIAA0971 | KIAA0971 protein |
| | Hs.28164 | -0.7 | -10.47 | 0.001 | | ESTs |
| | Hs.112644 | -0.64 | -3.02 | 0.001 | | ESTs |
| | Hs.203557 | -0.64 | -3.4 | 0.001 | | ESTs |
| | Hs.172825 | -0.58 | -3.86 | 0.001 | KIAA1037 | KIAA1037 protein |
| | Hs.90791 | -0.49 | -2.84 | 0.001 | GABRA6 | gamma-aminobutyric acid (GABA) A receptor, alpha 6 |
| | Hs.99642 | -0.49 | -1.58 | 0.001 | | ESTs |
| | Hs.112761 | -0.46 | -2.12 | 0.001 | | ESTs |
| | Hs.18724 | -0.46 | -2.33 | 0.001 | | Homo sapiens mRNA; cDNA DKFZp564F093 (from clone DKFZp564F093) |
| | Hs.283559 | -0.44 | -1.52 | 0.001 | | ESTs |
| | Hs.93502 | -0.39 | -1.59 | 0.001 | U1SNRNPBP | U1-snRNP binding protein homolog (70kD) |
| | Hs.179718 | -0.38 | -2.6 | 0.001 | MYBL2 | v-myb avian myeloblastosis viral oncogene homolog-like 2 |
| | Hs.6289 | -0.38 | -1.59 | 0.001 | | Homo sapiens cDNA: FLJ20886 fis, clone ADKA03257 |
| | Hs.81942 | -0.37 | -1.6 | 0.001 | POLA2 | polymerase (DNA-directed), alpha (70kD) |
| | Hs.182231 | -0.98 | -6.73 | 0.01 | TRH | thyrotropin-releasing hormone |
| | Hs.278658- | 0.97 | 2.85 | 0.01 | KRTHB6 | keratin, hair, basic, 6 (monilethrix) |
| | Hs.119571 | -0.96 | -4.83 | 0.01 | COL3A1 | collagen, type III, alpha 1 (Ehlers-Danlos syndrome type IV, autosomal dominant) |
| | Hs.27169 | -0.96 | -2.95 | 0.01 | | EST |

(continued)

| Drug | Unigene | r | slope | P value | Symbol | Description |
|---|---|---|---|---|---|---|
| | Hs.131118 | -0.95 | -2.42 | 0.01 | | ESTs, Moderately similar to CB80_HUMAN 80 KDA NUCLEAR CAP BINDING PROTEIN [H.sapiens] |
| | Hs.193124 | -0.93 | -6.44 | 0.01 | PDK3 | pyruvate dehydrogenase kinase, isoenzyme 3 |
| VCR | | | | | | |
| | Hs.136807 | 0.99 | 2.1 | 0.001 | 0 | ESTs |
| | Hs.117546 | 0.9 | 6.24 | 0.001 | NNAT | neuronatin |
| | Hs.6844 | 0.75 | 4.05 | 0.001 | FLJ20510 | hypothetical protein FLJ20510 |
| | Hs.133066 | 0.75 | 2.05 | 0.001 | 0 | ESTs |
| | Hs.131776 | 0.7 | 1.79 | 0.001 | FLJ20208 | hypothetical protein FLJ20208 |
| | Hs.77840 | -0.64 | -3.71 | 0.001 | ANXA4 | annexin A4 |
| | Hs.2256 | -0.63 | -4.87 | 0.001 | MMP7 | matrix metalloproteinase 7 (matrilysin, uterine) |
| | Hs.248109 | 0.63 | 2.08 | 0.001 | ETS1 | v-ets avian erythroblastosis virus E26 oncogene homolog 1 |
| | Hs.158203 | -0.6 | -3.19 | 0.001 | ABLIM | actin binding LIM protein 1 |
| | Hs.259785 | -0.6 | -1.97 | 0.001 | CPT1A | carnitine palmitoyltransferase I, liver |
| | Hs.54089 | 0.6 | 1.86 | 0.001 | BARD1 | BRCA1 associated RING domain 1 |
| | Hs.7773 | 0.59 | 2.51 | 0.001 | 0 | ESTs, Weakly similar to A4P_HUMAN INTESTINAL MEMBRANE A4 PROTEIN [H.sapiens] |
| | Hs.31773 | -0.59 | -1.5 | 0.001 | OKL38 | pregnancy-induced growth inhibitor |
| | Hs.78629 | -0.58 | -2.95 | 0.001 | ATP1B1 | ATPase, Na+/K+ transporting, beta 1 polypeptide |
| | Hs.9030 | 0.58 | 4.34 | 0.001 | TONDU | TONDU |
| | Hs.296842 | 0.57 | 1.74 | 0.001 | 0 | Smooth muscle myosin heavy chain isoform SMemb [human, umbilical cord, fetal aorta, mRNA Partial, 971 nt] |
| | Hs.25590 | 0.56 | 3.76 | 0.001 | STC1 | stanniocalcin 1 |
| | Hs.158203 | -0.55 | -2.37 | 0.001 | ABLIM | actin binding LIM protein 1 |
| | Hs.194710 | -0.55 | -4.25 | 0.001 | GCNT3 | glucosaminyl (N-acetyl) transferase 3, mucin type |
| | Hs.116462 | -0.54 | -3.32 | 0.001 | 0 | ESTs |
| VLB | | | | | | |
| | Hs.112788 | 0.83 | 1.62 | 0.001 | | EST |
| | Hs.122926 | -0.8 | -3.35 | 0.001 | | ESTs |
| | Hs.101337 | 0.76 | 1.88 | 0.001 | UCP3 | uncoupling protein 3 (mitochondrial, proton carrier) |
| | Hs.110736 | -0.72 | -3.52 | 0.001 | SLC12A2 | solute carrier family 12 (sodium/ potassium/chloride transporters), member 2 |
| | Hs.7089 | -0.62 | -1.5 | 0.001 | LOC51141 | insulin induced protein 2 |
| | Hs.155109 | -0.57 | -3.73 | 0.001 | HSD17B2 | hydroxysteroid (17-beta) dehydrogenase 2 |
| | Hs.104476 | -0.57 | -1.94 | 0.001 | | ESTs |
| | Hs.75285 | -0.54 | -2.4 | 0.001 | ITIH2 | inter-alpha (globulin) inhibitor, H2 polypeptide |
| | Hs.5724 | -0.53 | -1.75 | 0.001 | INADL | PDZ domain protein (Drosophila inaD-like) |
| | Hs.77840 | -0.51 | -3.58 | 0.001 | ANXA4 | annexin A4 |

(continued)

| Drug | Unigene | r | slope | P value | Symbol | Description |
|---|---|---|---|---|---|---|
| | Hs.115412 | -0.51 | -3.68 | 0.001 | | Homo sapiens cDNA FLJ13881 fis, clone THYRO1001458, moderately similar to MYOSIN HEAVY CHAIN, NONMUSCLE TYPE B |
| | Hs.8297 | -0.51 | -3.15 | 0.001 | RNASE6PL | ribonuclease 6 precursor |
| | Hs.83758 | 0.51 | 2.17 | 0.001 | CKS2 | CDC28 protein kinase 2 |
| | Hs.172182 | -0.51 | -1.86 | 0.001 | PABPC1 | poly(A)-binding protein, cytoplasmic 1 |
| | Hs.116462 | -0.49 | -3.76 | 0.001 | | ESTs |
| | Hs.2679 | -0.49 | -2.96 | 0.001 | GJB1 | gap junction protein, beta 1, 32kD (connexin 32, Charcot-Marle-Tooth neuropathy, X-linked) |
| | Hs.147049 | -0.49 | -1.51 | 0.001 | CUTL1 | cut (Drosophila)-like 1 (CCAAT displacement protein) |
| | Hs.107164 | -0.49 | -1.69 | 0.001 | SPTBN1 | spectrin, beta, non-erythrocytic 1 |
| | Hs.286035 | -0.49 | -1.79 | 0.001 | | Homo sapiens cDNA: FLJ22686 fis, clone HSI10987 |
| | Hs.93659 | -0.47 | -3.45 | 0.001 | ERP70 | protein disulfide isomerase related protein (calcium-binding protein, intestinal-related) |

Since the plasma concentrations of 5FU and MTX, both of which are metabolic antagonists, did not reach the levels in nude mice that they do in patients receiving clinical doses, it is difficult to evaluate further the sensitivity (T/C) of tumors to those two drugs. Therefore 1228 selected genes showing possible association with sensitivity to at least one of the remaining seven drugs were selected for further analysis. Of those 1228 genes, 333 revealed significant correlation with two or more drugs, suggesting that some common mechanisms may be involved in drug response. Seventeen genes appeared to be correlated with all seven drugs, and 15 genes with six. None of the 333 genes that showed correlation between expression level and response to two or more drugs revealed inverse results from one drug to another; i.e., if a gene showed a positive correlation with one drug, it also had a positive correlation(s) with other drugs, and similarly no exceptions were observed in the case of negative correlations. The 32 genes commonly associated with efficacy of six or seven drugs included *CCNB1* and *BUB1B;* and 895 others showed significant correlation with sensitivity to only one of the seven drugs, reflecting drug-specific chemosensitivity.

Among the 333 genes with response to multiple drugs, some whose expression levels correlated with more significance in xenografts derived from specific organs than in all 85 xenografts combined were detected. For instance, the expression level of *TNFRSF14,* a member 14 of the tumor necrosis factor receptor superfamily, showed a much stronger correlation with sensitivity to ACNU in 11 xenografts from non-small cell lung cancers (r=-0.87,p<0.001) than in all 85 xenografts combined (r=-0.42, *p*<0.001). Furthermore, compared with the correlation in xenografts from other tissues, it was clear that the expression level of *TNFRSF14* has almost no relation to sensitivity to ACNU in xenografts derived from breast or gastric cancers.

### Example 5

### Drug sensitivity score

To apply these gene subsets to clinical use, an algorithm to calculate "drug sensitivity score" using the value of expression levels of the sensitivity related genes to each anti-cancer drug was established. A significant correlation between the scores and the sensitivities to each of five anti-cancer drugs among 85 xenografts that revealed various responses to each anti-cancer drug was observed, indicating a possibility to establish a scoring system to predict the sensitivity to a particular anti-cancer drug using a set of genes.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be within the scope of the following claims.

**Claims**

1. A method of screening compounds, comprising

   a) providing

   i) an *in vitro* or xenograft cancer sample comprising a known drug sensitivity profile; wherein said drug sensitivity profile consists of information on the levels of expression of a each of a panel of genes correlated to sensitivity of cancer cells to cancer chemotherapy drugs when cancer cells are exposed to a panel of said chemotherapy drugs, wherein said panel of genes is selected from the group consisting of ADAMTS2, MSH6, MAGEA6, RPS6KB1, PIK3CB, MDR3, MDR4, MDR9, OS4, KIAA1140, GCNT3, FARP1, PP1044, C110RF15, KIAA0233, BZRP, PRPS1, HGFAC, TRPC4, LOC56889, FLJ20208, FLJ22833, PGLYRP, TNFRSF14, HLA-B, NPR3, DES, PCDH1, LLGL2, HMGCL, TSPAN, ABP1, ERP70, HSD17B2, FBP1, SIM1, LAF4, CRKL, TOB2, GDA, MMP7, PRSS8, CKS2, KIF4A, PABPC1, FLJ21865, ETV4, EXTL3, PIK3C2A, DNMT3B, DKFZP566G1424, KIAA0026, CCNB1, GPC3, EVA1, FSTL3, MSLN, FLJ21935, STK17A, KCNH2, DLK1, CPX, NRP1, PCTAIRE2BP, ELF3, AGR2, FLJ10849, ATP1B1, GPX2, SLI, AMPD1, OTC, MMP3, COX6A2, S100A4, LOC51315, MDS024, NK4, TRAF2, ARHE, LOC51256, ITGA3, KIAA0971, KIAA1037, GABRA6, U1SNRNPBP, MYBL2, POLA2, TRH, KRTHB6, COL3A1, PDK3, NNAT, FLJ20510, ANXA4, ETS1, ABLIM, CPT1A, BARD1, OKL38, TONDU, STC1, UCP3, SLC12A2, LOC51141, ITIH2, INADL, RNASE6PL, GJB1, CUTL1, SPTBN1, FLJ13881, LGALS4, C11ORF9, ARSE, LAMB3, MVP, UGT1A1, GLJ20053, CD74, BUB1B, CCND1, CCNE1, TOP2A, TYMS, ALDH1, and CYP3A5; and
   ii) a test compound

   b) exposing said cancer sample to said test compound;
   c) determining the effect of said test compound on the level of expression of of each of said drug sensitivity genes in said cancer sample;
   d) comparing the effect of said test compound on the level of expression of each of said drug sensitivity genes in said cancer sample to said drug sensitivity profile; and
   e) classifying said test compound according to whether the expression levels of one or more of said drug sensitivity genes alter in response to said test compound in the same manner as the expression levels of the same one or more drug sensitivity genes alter in response to at least one of said known cancer chemotherapy drugs.

2. The method of Claim 1, wherein said panel of cancer chemotherapeutic drugs is selected from the group consisting of 5FU, ACNU, ADR, CPM, DDP, MMC, MTX, VCR, and VLB.

3. The method of Claim 1, wherein said test compound is a candidate cancer chemotherapeutic.

4. The method of Claim 1, wherein said detecting the level of expression of said drug sensitivity genes comprises detecting the level of mRNA expressed from each of said drug sensitivity genes.

5. The method of Claim 4, wherein said detecting the level of mRNA expressed from said drug sensitivity genes comprises exposing said mRNA to a nucleic acid probe complementary to said mRNA.

6. The method of Claim 4, wherein said detecting the level of mRNA expressed from said drug sensitivity genes comprises performing an INVADER assay.

7. The method of Claim 1, wherein said detecting the level of expression of said drug sensitivity genes comprises detecting the level of polypeptide expressed from each of said drug sensitivity genes.

8. The method of Claim 7, wherein said detecting the level of polypeptide expressed from said drug sensitivity genes comprises exposing said polypeptide to an antibody specific to said polypeptide and detecting the binding of said antibody to said polypeptide.

9. The method of Claim 1, wherein said method further comprises the step of selecting a composition for use as a medicament, wherein said expression levels of one or more of said drug sensitivity genes indicate sensitivity of said cancer sample to said test compound.

10. The method of Claim 1, wherein said determining the effect of said test compound on the level of expression of each of said drug sensitivity genes in said cancer sample comprises detecting the level of mRNA expressed from said drug sensitivity genes.

11. The method of Claim 10, wherein said detecting the level of mRNA expressed from said drug sensitivity genes in said cancer sample comprises exposing said mRNA to a nucleic acid probe complementary to said mRNA.

12. The method of Claim 10 wherein said detecting the level of mRNA expressed from said drug sensitivity genes in said cancer sample comprises performing an INVADER assay.

13. The method of Claim 1, wherein said determining the effect of said test compound on the level of expression of each of said drug sensitivity genes in said cancer sample comprises detecting the level of polypeptide expressed from said drug sensitivity genes.

14. The method of Claim 13, wherein said determining the effect of said test compound on the level of expression of each of said drug sensitivity genes in said cancer sample comprises exposing said polypeptide to an antibody specific to said polypeptide and detecting the binding of said antibody to said polypeptide.

**European Patent Office** | **EUROPEAN SEARCH REPORT** | Application Number: EP 08 00 7724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCHERF U ET AL: "A gene expression database for the molecular pharmacology of cancer" NATURE GENETICS, NATURE AMERICA, NEW YORK, US, vol. 24, no. 3, 1 March 2000 (2000-03-01), pages 236-244, XP002224798 ISSN: 1061-4036 * the whole document * | 1-14 | INV. C12Q1/68 |
| X | STAUNTON J E ET AL: "Chemosensitivity prediction by transcriptional profiling" PROCEEDINGS OF THE NATIONAL ACADEMY OF SCIENCES OF USA, NATIONAL ACADEMY OF SCIENCE, WASHINGTON, DC, vol. 98, no. 19, 11 September 2001 (2001-09-11), pages 10787-10792, XP002224800 ISSN: 0027-8424 * the whole document * | 1-14 | |
| X | WO 00/39336 A (ROSETTA INPHARMATICS INC [US]) 6 July 2000 (2000-07-06) * the whole document * | 1-14 | TECHNICAL FIELDS SEARCHED (IPC) C12Q |
| P,X | ZEMBUTSU H ET AL: "Genome-wide cDNA microarray screening to correlate gene expression profiles with sensitivity of 85 human cancer xenografts to anticancer drugs" CANCER RESEARCH, AMERICAN ASSOCIATION FOR CANCER RESEARCH, BALTIMORE, MD, vol. 62, no. 2, 15 January 2002 (2002-01-15), pages 518-527, XP002224797 ISSN: 0008-5472 * the whole document * | 1-14 | |

-/--

The present search report has been drawn up for all claims

| Place of search | Date of completion of the search | Examiner |
|---|---|---|
| The Hague | 27 June 2008 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**EUROPEAN SEARCH REPORT**

Application Number

EP 08 00 7724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | UEYAMA YOSHITO ET AL: "In vivo chemosensitivity of human malignant cystosarcoma phyllodes xenografts." ONCOLOGY REPORTS, vol. 7, no. 2, March 2000 (2000-03), pages 257-260, XP008021860 ISSN: 1021-335X * the whole document * | 1-14 | |
| Y | GREEN A R ET AL: "Comparisons of the effects of tamoxifen, toremifene and raloxifene on enzyme induction and gene expression in the ovariectomised rat uterus." JOURNAL OF ENDOCRINOLOGY, vol. 170, no. 3, September 2001 (2001-09), pages 555-564, XP002254037 ISSN: 0022-0795 * figures 1-5; table 2 * | 1-14 | |
| Y | HILSENBECK S G ET AL: "Statistical analysis of array expression data as applied to the problem of tamoxifen resistance." JOURNAL OF THE NATIONAL CANCER INSTITUTE. UNITED STATES 3 MAR 1999, vol. 91, no. 5, 3 March 1999 (1999-03-03), pages 453-459, XP009002755 ISSN: 0027-8874 * the whole document * | 1-14 | **TECHNICAL FIELDS SEARCHED (IPC)** |

-/--

The present search report has been drawn up for all claims

| Place of search | Date of completion of the search | Examiner |
|---|---|---|
| The Hague | 27 June 2008 | Botz, Jürgen |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................

& : member of the same patent family, corresponding
document

EPO FORM 1503 03.82 (P04C01)

**European Patent Office**

**EUROPEAN SEARCH REPORT**

Application Number

EP 08 00 7724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TAKECHI T ET AL: "Screening of differentially expressed genes in 5-fluorouracil-resistant human gastrointestinal tumor cells." JAPANESE JOURNAL OF CANCER RESEARCH: GANN. JAPAN JUN 2001, vol. 92, no. 6, June 2001 (2001-06), pages 696-703, XP002254038 ISSN: 0910-5050 * the whole document * | 1-14 | |
| Y | BEYER-SEHLMEYER G ET AL: "Suppressive subtractive hybridisation reveals differential expression of serglycin, sorcin, bone marrow proteoglycan and prostate-tumour-inducing gene I (PTI-1) in drug-resistant and sensitive tumour cell lines of haematopoetic origin." EUROPEAN JOURNAL OF CANCER, vol. 35, no. 12, November 1999 (1999-11), pages 1735-1742, XP002254039 ISSN: 0959-8049 * the whole document * | 1-14 | |
| Y | COLLIE-DUGUID E S R ET AL: "Cloning and initial characterization of the human DPYD gene promoter." BIOCHEMICAL AND BIOPHYSICAL RESEARCH COMMUNICATIONS, vol. 271, no. 1, 29 April 2000 (2000-04-29), pages 28-35, XP002255521 ISSN: 0006-291X * the whole document * | 1-14 | TECHNICAL FIELDS SEARCHED (IPC) |
| Y,D | US 5 846 717 A (OLIVE DAVID MICHAEL ET AL) 8 December 1998 (1998-12-08) * the whole document * | 6 | |

The present search report has been drawn up for all claims

| Place of search | Date of completion of the search | Examiner |
|---|---|---|
| The Hague | 27 June 2008 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 00 7724

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2008

| Patent document cited in search report | | Publication date | Patent family member(s) | | Publication date |
|---|---|---|---|---|---|
| WO 0039336 | A | 06-07-2000 | AU | 2377000 A | 31-07-2000 |
| | | | CA | 2356648 A1 | 06-07-2000 |
| | | | EP | 1141410 A1 | 10-10-2001 |
| | | | JP | 2003520567 T | 08-07-2003 |
| | | | US | 6801859 B1 | 05-10-2004 |
| US 5846717 | A | 08-12-1998 | US | 6001567 A | 14-12-1999 |
| | | | US | 5843669 A | 01-12-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 34695202 P
- US 4683195 A
- US 4683202 A
- US 4965188 A
- US 5846717 A
- US 6090543 A
- US 6001567 A
- US 5985557 A
- US 5994069 A
- US 5962233 A
- US 5538848 A
- US 5639606 A
- US 5643765 A
- US 5876978 A
- US 5885530 A
- US 4981785 A
- US 6159750 A
- US 5358691 A
- US 5599677 A
- US 5672480 A
- US 6198107 B
- US 4323546 A
- US 5223409 A
- US 5631169 A, Lakowicz
- US 4968103 A, Stavrianopoulos
- US 4109496 A
- US 5283317 A
- WO 9410300 A, Brent
- WO 0198537 A2
- US 5539082 A
- US 5714331 A
- US 5719262 A
- US 5489677 A
- US 5602240 A
- US 5034506 A
- US 3687808 A
- WO 0012738 A
- WO 0009675 A
- US 6033908 A
- US 6019978 A
- US 6001557 A
- US 5994132 A
- US 5994128 A
- US 5994106 A
- US 5981225 A
- US 5885808 A
- US 5872154 A
- US 5830730 A
- US 5824544 A
- US 9902685 W
- US 6180370 B
- US 5585089 A
- US 6054297 A
- US 5565332 A
- US 5705188 A
- WO 9730731 A
- US 4873191 A
- US 6080912 A
- WO 9008832 A
- WO 1990 A
- US 5614396 A

### Non-patent literature cited in the description

- **FURUKAWA et al.** *Clin Cancer Res.,* 1995, vol. 1, 305-11
- **SALONGA et al.** *Clin Cancer Res.,* 2000, vol. 6, 1322-7
- **UEDA et al.** *J Biol Chem.,* 1987, vol. 262, 505-8
- **TURTON et al.** *Oncogene,* 2001, vol. 20, 1300-6
- **ROSS et al.** *Nat Genet,* 2000, vol. 24, 227-35
- **SCHERF et al.** *Nat Genet,* 2000, vol. 24, 236-44
- **TASHIRO et al.** *Cancer Chemother Pharmacol,* 1989, vol. 24, 187-92
- **INABA et al.** *Jpn J Cancer Res.,* 1988, vol. 79, 517-22
- **MARUO et al.** *Anticancer Res,* 1990, vol. 10, 209-12
- **INABA et al.** *Cancer,* 1989, vol. 64, 1577-82
- **TASHIRO et al.** *Cancer Chemother Pharmacol.,* 1989, vol. 24, 187-92
- **TAHARA.** *Cancer,* 1995, vol. 75, 1410-7
- **MACKAY et al.** *Ultrastruct Pathol,* 1989, vol. 13, 561-71
- **ALIZADEH.** *Nature,* 2000, vol. 403, 503-11
- **CHEN et al.** *Cell,* 1986, vol. 47, 381-9
- **KAWANAMI et al.** *Oncol Res,* 1996, vol. 8, 197-206

- **RAMACHANDRAN et al.** *Biochem Pharmacol,* 1993, vol. 45, 1367-71
- **JOHNSTON et al.** *Cancer Res.,* 1995, vol. 55, 1407-12
- **CHEN ; WAXMAN.** *Biochem Pharmacol,* 1995, vol. 49, 1691-701
- **KITAZONO et al.** *J Natl Cancer Inst.,* 1999, vol. 91, 1647-53
- **KITAZONO et al.** *J Natl Cancer Inst,* 1999, vol. 91, 1647-53
- **SCHROEIJERS et al.** *Cancer Res.,* 2000, vol. 60, 1104-10
- **SCHROEIJERS et al.** *Am J Pathol,* 1998, vol. 152, 373-8
- **HUANG et al.** *Drug Metab Dispos,* 1996, vol. 24, 899-905
- **SCHUETZ et al.** *Mol Pharmacol.,* 1996, vol. 49, 311-8
- **SANTOS et al.** *Clin Cancer Res.,* 2000, vol. 6, 2012-20
- **INNOCENTI et al.** *Drug Metab Dispos,* 2001, vol. 29, 596-600
- **KONDOH et al.** *Cancer Res.,* 1999, vol. 59, 4990-6
- **LOTAN et al.** *Carbohydr. Res,* 1991, vol. 213, 47-57
- **BORST et al.** *J Natl Cancer Inst,* 2000, vol. 92, 1295-302
- **SUGIYAMA et al.** *Cancer Res.,* vol. 59, 4406-12
- **DAVENPORT et al.** *Genomics,* 1999, vol. 55, 113-7
- **BARLOGIE ; DREWINKO.** *Eur J Cancer,* 1978, vol. 14, 741-5
- **RAO ; RAO.** *J Natl Cancer Inst,* 1976, vol. 57, 1139-43
- **LEYLAND-JONES et al.** *Cancer Res.,* 1991, vol. 51, 587-94
- **KORNMANN et al.** *Cancer Res.,* 1999, vol. 59, 3505-11
- **SMITH ; SEO.** *Anticancer Res,* 2000, vol. 20, 2537-9
- **KACIAN et al.** *Proc. Natl. Acad. Sci. USA,* 1972, vol. 69, 3038
- **CHAMBERLIN et al.** *Nature,* 1970, vol. 228, 227
- **WU ; WALLACE.** *Genomics,* 1989, vol. 4, 560
- **SAMBROOK et al.** Molecular Cloning: A Laboratory Manual. Cold Spring Harbor Press, 1989, 9.31-9.58
- **SUMERDON et al.** *Nucl. Med. Biol,* 1990, vol. 17, 247-254
- **GRIFFIN et al.** *J Clin Onc,* 1991, vol. 9, 631-640
- **LAUFFER.** *Magnetic Resonance in Medicine,* 1991, vol. 22, 339-342
- **KHAW et al.** *Science,* 1980, vol. 209, 295
- **SCHEINBERG et al.** *Science,* 1982, vol. 215, 1511
- **HNATOWICH et al.** *Int. J. Appl. Radiat. Isot,* 1982, vol. 33, 327
- **WONG et al.** *Int. J. Appl. Radiat. Isot,* 1978, vol. 29, 251
- **WONG et al.** *J. Nucl. Med.,* 1981, vol. 23, 229
- **KOEHLER ; MILSTEIN.** *Nature,* 1975, vol. 256, 495
- **ZUCKENNANN et al.** *J. Med. Chem.,* 1994, vol. 37, 2678-85
- **LAM.** *Anticancer Drug Des,* 1997, vol. 12, 145
- **DEWITT et al.** *Proc. Natl. Acad. Sci. U.S.A.,* 1993, vol. 90, 6909
- **ERB et al.** *Proc. Nad. Acad. Sci. USA,* 1994, vol. 91, 11422
- **ZUCKERINANN et al.** *J. Med. Chem.,* 1994, vol. 37, 2678
- **CHO et al.** *Science,* 1993, vol. 261, 1303
- **CARRELL et al.** *Angew. Chem. Int. Ed. Engl,* 1994, vol. 33, 2059
- **CARELL et al.** *Angew. Chem. Int. Ed. Engl,* 1994, vol. 33, 2061
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc. Nad. Acad. Sci. USA,* 1992, vol. 89, 18651869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci,* 1990, vol. 87, 6378-6382
- **FELICI.** *J. Mol. Biol.,* 1991, vol. 222, 301
- **MCCONNELL et al.** *Science,* 1992, vol. 257, 1906-1912
- **SJOLANDER ; URBANICZKY.** *Anal. Chem.,* 1991, vol. 63, 2338-2345
- **SZABO et al.** *Curr. Opin. Struct. Biol,* 1995, vol. 5, 699-705
- **RIVAS ; MINTON.** *Trends Biochem Sci,* 1993, vol. 18, 284-7
- Current Protocols in Molecular Biology. J. Wiley, 1999
- **HEEGAARD.** *J. Mol. Recognit,* 1998, vol. 11, 141-8
- **HAGEAND TWEED.** *J. Chromatogr. Biomed. Sci. Appl,* 1997, vol. 699, 499-525
- **ZERVOS et al.** *Cell,* 1993, vol. 72, 223-232
- **MADURA et al.** *J. Biol. Chem.,* 1993, vol. 268, 12046-12054
- **BARTEL et al.** *Biotechniques,* 1993, vol. 14, 920-924
- **IWABUCHI et al.** *Oncogene,* 1993, vol. 8, 1693-1696

- **GHOSE et al.** *Methods Enzymol.,* 1983, vol. 93, 280
- **BRINSTER et al.** *Proc. Natl. Acad. Sci. USA,* 1985, vol. 82, 4438-4442
- **JANENICH.** *Proc. Natl. Acad. Sci. USA,* 1976, vol. 73, 1260
- **HOGAN et al.** Manipulating the Mouse Embryo. Cold Spring Harbor Laboratory Press, 1986
- **JAHNER et al.** *Proc. Natl. Acad Sci. USA,* 1985, vol. 82, 6927
- **STEWART et al.** *EMBO J.,* 1987, vol. 6, 383
- **JAHNER et al.** *Nature,* 1982, vol. 298, 623
- **HASKELL ; BOWEN.** *Mol. Reprod. Dev,* 1995, vol. 40, 386
- **EVANS et al.** *Nature,* 1981, vol. 292, 154
- **BRADLEY et al.** *Nature,* 1984, vol. 309, 255
- **GOSSLER et al.** *Proc. Acad. Sci. USA,* 1986, vol. 83, 9065
- **ROBERTSON et al.** *Nature,* 1986, vol. 322, 445
- **INABA et al.** *Jpn J Cancer Res.,* 1988, vol. 79, 517
- **OHNISHI, Y.** Anticancer Drug Sensitivities of Human Tumors Transplanted. *The Nude Mouse and Anticancer Drug Evaluation,* 1996, 219-223
- **ONO et al.** *Cancer Res.,* 2000, vol. 60, 5007